# EUROPEAN PATENT APPLICATION

(11) **EP 4 299 734 A1**
(43) Date of publication of application: **03.01.2024**
(21) Application number: 22182675.3
(22) Date of filing: 01.07.2022
(51) Int. Cl.: C12N 9/22, C12N 15/113, C07K 1/04, C07K 14/47, C07K 14/74, C12N 15/10, G01N 33/48, G01N 33/50

(54) **CELL LINE FOR DISCOVERING TCR ANTIGENS AND USES THEREOF**

(71) Applicant: ETH Zurich, 8092 Zurich (CH)
(72) Inventor: HONG, Kai-Lin, 4058 Basel (CH); KUCHARCZYK, Jakub, 4054 Basel (CH); VAZQUEZ-LOMBARDI, Rodrigo, 4125 Riehen (CH); REDDY, T, Sai, 4051 Basel (CH)
(74) Representative: Vossius & Partner Patentanwälte Rechtsanwälte mbB

(57) **Abstract**

The present invention relates to a cell line wherein the endogenous class I and/or class II HLA alleles are inactivated, the cell line further comprising (a) a polynucleotide encoding a first fluorescent marker undercontrol of at least one STAT response element, and (b) an interleukin 2 (IL-2) receptor. The invention further relates to the use of said cell line for the identification of antigenic peptide and/or the identification of alloreactive T cell receptors.

## Description

A promising strategy for immunotherapy of solid tumours is the development of genetically engineered T cells. In this approach, autologous patient T cells are isolated, genetically modified to express a tumour-targeting receptor and reinfused into patients. In contrast to immune checkpoint blockade, in which T cells are activated indiscriminately, engineered T cell therapies provide control over the number of infused T cells, as well as their phenotype, their tumour-specific receptor and the targeted tumour antigen (i.e., targeted immunotherapy). The most clinically advanced modality of engineered T cells are chimeric antigen receptor (CAR) T cells, in which T cells are engineered to express an antibody-based synthetic receptor targeting surface tumour antigens. There are currently five approved CAR-T cell therapies targeting CD19 for the treatment of advanced B cell leukaemia and lymphomas, some of which have shown unprecedented complete response rates of ~90% in the setting of treatment-refractory cancers. Despite their remarkable success in liquid cancers, CAR-T cells have shown disappointing efficacy against solid tumours, with recent clinical trials showing low overall response rates (~ 10% on average in trials with more than 10 patients). An emerging modality of engineered T cells, namely TCR-redirected T cells or TCR-T, has shown significant promise for the treatment of solid tumours due to substantially improved overall response rates across several advanced cancer indications (approximately 35% on average for trials with more than 10 patients). Similar to CAR T cells, TCR-T cells are currently generated by viral transduction of autologous patient T cells, followed by their expansion and re-infusion. However, instead of antibody-based chimeric receptors, T cells are transduced with constructs encoding the alpha and beta chains of tumour-reactive TCRs. Different to CARs, TCRs can recognize intracellular antigen targets in the form of peptides bound to multiple histocompatibility complexes (MHC), thus vastly increasing the targetable antigen pool. This is an attractive property for the treatment of solid tumours, for which highly specific surface antigens (i.e., CAR T cell targets) have been difficult to identify. Additional properties that may explain the enhanced activity of TCR-T cells against solid tumours relative to CAR-T cells include more efficient signalling by the TCR complex and moderate affinity of TCRs to their peptide-MHC targets, resulting in lower levels of T cell exhaustion and enhanced ability for serial triggering.

Different from antibodies, which are highly specific and typically recognize a single epitope, a large proportion of TCRs are able to recognize multiple peptide antigens (presented by MHC, or HLA in humans). This naturally occurring TCR promiscuity is thought to maximise the number of potential pathogen-derived or tumour peptides that can trigger a T cell response. Thus, TCR cross-reactivity appears to fulfil an important physiological role in immunity, for example, naturally-occurring TCRs have been shown to display a wide range of specificity profiles, with some TCRs estimated to recognize up to a million different peptides. In addition to cross-reactivity, some TCRs have the potential to be alloreactive, meaning that they can recognise a particular HLA allele irrespective of which peptide is presented. Thus, it is crucial to carefully profile TCR cross-reactivity and alloreactivity in order to develop safe TCR-based therapies.

Traditional methods for the assessment of TCR cross-reactivity (off-target activity) include the co-culture of TCR-T wells with large panels of human primary cells and, more recently, with panels of induced pluripotent stem cells (iPSCs) shortly after differentiation. While essential, these methods are time-consuming and expensive, and in some cases not sufficiently accurate to predict potentially dangerous TCR off-targets. In this context, the application of peptide antigen scanning for TCR cross-reactivity profiling has been a useful recent addition to the safety screening toolbox of therapeutic TCR development. This method consists in the co-culture of TCR-T cells with APCs that are pulsed with peptide antigen variants, typically single-amino acid variants tiled across the target peptide. TCR-T activation data is used to derive peptide motifs at different activation thresholds, and resulting motifs are then used to interrogate human proteome databases. The result is the prediction of potential TCR off-targets in the human proteome, which are then used in subsequent safety screening steps. Despite its usefulness, peptide scanning remains a low-throughput method that carries significant cost due to the requirement of purchasing individual synthetic peptides for pulsing of APCs (one peptide per co-culture well assay) as opposed to a screening in a pooled fashion. This typically limits the number of peptides that can be screened to a few hundred. In terms of alloreactivity, a commonly used method consists in the co-culture of TCR-T cells with EBV-immortalised B-cell lymphoblastoid cell lines (B-LCL). This is mostly due to the availability of hundreds of HLA-typed B-LCLs collected and maintained by the International Histocompatibility Working Group (IHWG). Similar to peptide scanning, this is a low-throughput method that is further limited by the occurrence of EBV-reactivity in the great majority of T cell donors, thus leading to high levels of background activation and a low signal-to-noise ratio. In fact, particular B-LCLs, presumably those presenting large amounts of EBV peptides on their surface, are unsuitable for alloreactivity screens due to excessively high background levels.

A number of higher throughput technologies with applications in T cell antigen discovery and TCR cross-reactivity profiling have been developed over the past 5 years. These methods can be classified into those that rely on receptor binding and those relying on cellular function. In the former class of technologies, fluorescently-labelled peptide-MHC multimer complexes are commonly used to identify antigen-specific T cells by fluorescence-activated cell sorting (FACS). There has been substantial innovation in this area in recent years, particularly with the development of DNA-barcoded peptide-MHC multimers that allow for simultaneous determination of both TCR and antigen identity via deep sequencing. However, one major limitation of binding-based T cell antigen discovery platforms is that low-affinity antigen-specific T cells often fail to be detected by peptide-MHC multimer staining protocols. This is particularly relevant to the discovery of TCRs recognising self-tumour antigens, which often display low affinities. More importantly, these platforms are unable to fully exclude TCR cross-reactivity, especially when considering that TCR toxicity can occur even at low affinities to off-targets. Another limitation of multimer-based technologies relates to the small library sizes that can be feasibly generated. Such libraries typically consist of 100-1000 peptides, as peptide-MHC multimers need to be assembled individually in order to preserve the information linking the DNA barcode and displayed peptide. Examples of libraries that have been generated in the peptide-MHC multimer format include positional scanning libraries (~ 191 peptides, (Bentzen et al., Nat Biotechnol. 2018 Nov 19. doi: 10.1038/nbt.4303.) and putative neoantigen libraries derived from tumour exome sequencing information (315 peptides, (Zhang et al., Nat Biotechnol. 2018 Nov 12;10.1038/nbt.4282). The library size limitation of binding-based technologies has been addressed with the recent development of yeast display platforms supporting synthetic peptide-MHC library; by genetically encoding the peptide libraries in yeast cells coupled with deep sequencing, diversities of ten to a hundred million can be screened (Gee et al., Cell. 2018 Jan 25;172(3):549-563.e16). In this method, candidate TCRs (e.g., TCRs from tumour-infiltrating lymphocytes (TIL)) are modified for recombinant expression as soluble TCR tetramers that can be used to stain yeast cells displaying peptide-MHC libraries. Yeast cells that bind TCR tetramers are enriched by several rounds of FACS and their peptide library locus sequenced. Using this information, the synthetic peptide binding profiles of selected TCRs are determined and used to generate predictions of human peptide antigens. Interestingly, a subset of human peptide antigens predicted using this technology are not able to trigger signalling in T cell activation assays. This observation further highlights an important disconnect between binding (the basis of yeast display) and function (T cell activation) in the context of TCR:peptide-MHC interactions.

Technologies relying on T cell function for antigen discovery and cross-reactivity profiling provide several advantages over methods relying on peptide-MHC binding alone. A notable advantage of functional TCR antigen screening platforms is their enhanced sensitivity, which results from the fact that T cells typically require low levels of displayed antigen for activation. A first group of recently reported technologies rely on the incorporation of chimeric MHC-TCR (Kisielow et al., 2019 May;20(5):652-662) or MHC-CD3 (Joglekar et al., Nat Methods. 2019 Feb;16(2):191-198) receptors, which allow for detection of signalling in reporter APCs upon peptide-MHC engagement by antigen-specific TCRs. A second type of technology relies on the process of trogocytosis (Li et al., Nat Methods. 2019 Feb;16(2):183-190), a naturally occurring phenomenon in which cell membrane fragments are spontaneously transferred from an antigen-specific T cell to an APC displaying its cognate peptide. A third group of technologies rely on the detection of soluble factors secreted by activated T cells by the stimulating APC. These include platforms harbouring functional reporters of granzyme activity (Kula et al., Cell. 2019 Aug 8;178(4):1016-1028.e13; Sharma et al., Nat Commun. 2019 Oct 7;10(1):4553) or expressing membrane-tethered antibodies for the capture of secreted interleukin-2 (IL-2) following T cell activation (Lee and Meyerson, Sci Immunol. 2021 Jan 22;6(55):eabf4001). While they have provided an important step forward, most of these platforms still rely on biased libraries (e.g., neoantigens) with diversities well below 10⁴ or utilise chimeric MHC-based receptors in which peptides are not presented in their natural context (e.g. covalently linked to MHC). Furthermore, all of them rely on lentiviral or retroviral transduction of candidate T cell antigen libraries which can be limited by random integration, heterogeneous expression and potential multicopy integration of library transgenes.

There is thus still a need in the art for cell lines that allow the presentation of TCR antigens and/or HLA alleles to T cells in a controlled manner. Further, there is a need in that art for methods that allow for the high throughput screening of TCR antigen libraries or HLA allele libraries.

Accordingly, it is an objective of the present invention to provide a cell line that allows for the controlled presentation of TCR antigens and/or HLA alleles.

It is a further objective of the present invention to provide improved methods for functional identification of TCR:peptide-MHC interactions in a high throughput manner.

These and other objectives are achieved by the independent claims of the present invention. The dependent claims are related to specific embodiments.

### SUMMARY OF THE INVENTION

The present invention is characterized in the herein provided embodiments and claims. In particular, the present invention relates, *inter alia,* to the following embodiments:
1. A cell line wherein the endogenous class I and/or class II HLA alleles are inactivated, the cell line further comprising
   a) a polynucleotide encoding a first fluorescent marker under control of at least one STAT response element, and
   b) an interleukin 2 (IL-2) receptor.
2. The cell line according to embodiment 1, wherein the polynucleotide encoding the first fluorescent marker is under control of at least 2, 3, 4 or 5 STAT response elements.
3. The cell line according to embodiment 1 or 2, wherein the IL-2 receptor is an engineered IL-2 receptor, in particular wherein the engineered IL-2 receptor comprises an engineered common gamma chain.
4. The cell line according to any one of embodiments 1 to 3, wherein the cell line further comprises a polynucleotide encoding a sequence-specific endonuclease, in particular wherein the sequence-specific endonuclease is a CRISPR-associated (Cas) protein, in particular wherein the CRISPR-associated (Cas) protein is Cas9.
5. The cell line according to any one of embodiments 1 to 4, wherein the cell line further comprises a heterologous polynucleotide encoding an HLA allele.
6. The cell line according to embodiment 5, wherein the endogenous class I HLA alleles are inactivated in said cell line and wherein the cell line comprises a heterologous polynucleotide encoding a class I HLA allele.
7. The cell line according to any one of embodiments 1 to 6, wherein the endogenous gene encoding beta-2 microglobulin is inactivated in said cell line.
8. The cell line according to any one of embodiments 1 to 7, wherein the cell line further comprises a heterologous polynucleotide encoding a beta-2 microglobulin.
9. The cell line according to embodiment 8, wherein the cell line further comprises a polynucleotide encoding a peptide, preferably wherein the polynucleotide encoding the beta-2 microglobulin and the polynucleotide encoding the peptide are transcriptionally fused.
10. The cell line according to embodiment 9, wherein the peptide is an MHC class I peptide.
11. The cell line according to embodiment 9 or 10, wherein the polynucleotide encoding the beta-2 microglobulin and the polynucleotide encoding the peptide are fused via a linker.
12. The cell line according to embodiment 11, wherein the linker encodes a protease-specific cleavage site and/or a self-cleaving peptide.
13. The cell line according to any one of embodiments 9 to 12, wherein the peptide further comprises a signal peptide.
14. A method for identifying potential off-targets of a T cell receptor (TCR), the method comprising the steps of:
   a) providing a plurality of cells according to any one of embodiments 9 to 13, wherein at least two cells comprised in the plurality of cells encode a different peptide variant that has been obtained by mutagenesis of a known antigenic peptide;
   b) contacting the plurality of cells of step (a) with a plurality of T cells encoding a TCR that is specific for said known antigenic peptide;
   c) isolating cells that express the first fluorescent marker;
   d) sequencing the polynucleotides encoding the peptide variants in the cells that have been isolated in step (c); and
   e) identifying potential off-targets of the TCR of interest based on the sequencing results that have been obtained in step (d).
15. The method according to embodiment 14, wherein the plurality of cells encode at least 5, 10, 20, 50, 100, 200, 300, 500 or 1000 different peptide variants that have been obtained by mutagenesis of a known antigenic peptide.
16. The method according to embodiment 14 or 15, wherein the peptide variants have been obtained by site-directed mutagenesis of the known antigenic peptide, in particular by site-directed saturation mutagenesis of the known antigenic peptide.
17. The method according to any one of embodiments 14 to 16, wherein the cells that express the first fluorescent marker are isolated by fluorescence-activated cell sorting (FACS).
18. The method according to any one of embodiments 14 to 17, wherein the polynucleotides encoding the peptide variants are sequenced by Sanger sequencing.
19. The method according to any one of embodiments 14 to 17, wherein the polynucleotides encoding the peptide variants are sequenced by deep sequencing.
20. The method according to embodiment 19, wherein potential off-targets are identified by read enrichment analysis of the deep sequencing results.
21. The method according to any one of embodiments 14 to 20, the method comprising an additional step of querying a potential off-target of a TCR that has been identified in step (e) against a protein database.
22. A method for identifying a target of a T cell receptor (TCR) of interest, the method comprising the steps of:
   a) providing a plurality of cells according to any one of embodiments 9 to 13, wherein at least two cells comprised in the plurality of cells encode a different peptide candidate;
   b) contacting the plurality of cells of step (a) with a plurality of T cells encoding a TCR of interest;
   c) isolating cells that express the first fluorescent marker;
   d) sequencing the nucleic acids encoding the peptide candidates in the cells that have been isolated in step (c); and
   e) identifying a target of the TCR of interest based on the sequencing results that have been obtained in step (d).
23. The method according to embodiment 22, wherein the plurality of cells encode at least 5, 10, 20, 50, 100, 200, 300, 500, 1'000, 10'000, 100'000 or 1'000'000 different peptide candidate.
24. The method according to embodiment 22 or 23, wherein the cells that express the first fluorescent marker are isolated by fluorescence-activated cell sorting (FACS).
25. The method according to any one of embodiments 22 to 24. wherein the polynucleotides encoding the peptide candidates are sequenced by Sanger sequencing.
26. The method according to any one of embodiments 22 to 25, wherein the polynucleotides encoding the peptide candidates are sequenced by deep sequencing.
27. The method according to embodiment 26, wherein potential targets of the TCR of interest are identified by read enrichment analysis of the deep sequencing results.
28. The method according to embodiment 26 or 27, the method comprising a further step of predicting targets of the TCR of interest by applying a machine learning model to a human peptidome database, wherein the machine learning model has been trained with the deep sequencing data.
29. A method for assessing the alloreactivity of a T cell receptor (TCR), the method comprising the steps of:
   a) providing a plurality of cells according to embodiment 5 or 6, wherein the plurality of cells encode at least one heterologous HLA allele;
   b) contacting the plurality of cells of step (a) with a plurality of T cells;
   c) isolating cells that express the first fluorescent marker;
   d) sequencing the heterologous polynucleotides encoding the HLA alleles in the cells that have been isolated in step (c); and
   e) identifying an HLA molecule as a target of an alloreactive TCR based on the sequencing results that have been obtained in step (d).
30. The method according to embodiment 29, wherein at least two cells in the plurality of cells of step (a) encode a different HLA allele.
31. The method according to embodiment 29 or 30, wherein at least 5, 10, 25, 50, 75, 100, 150, 200, 300, 400, 500, 1'000, 2'500, 5'000, 10'000 or 25'000 cells in the plurality of cells of step (a) encode a different HLA allele.
32. The method according to any one of embodiments 29 to 31, wherein the T cells express an identical TCR.
33. The method according to any one of embodiments 29 to 31, wherein at least two T cells in the plurality of T cells express different TCRs.
34. The method according to embodiment 33, wherein at least 5, 10, 20, 30, 40, 50, 60, 70, 80, 90 or 100 T cells in the plurality of T cells express different TCRs.
35. The method according to any one of embodiments 29 to 34, wherein the T cell is an engineered T cell, in particular wherein the engineered T cell comprises a polynucleotide encoding a second fluorescent marker under control of an NFAT transcription factor.
36. The method according to embodiment 35, the method comprising further steps of
   f) isolating T cells that express the second fluorescent marker;
   g) sequencing the polynucleotides encoding the TCRs in T cells that have been isolated in step (f); and
   h) identifying a TCR as an alloreactive TCR based on the sequencing results that have been obtained in step (g).
37. The method according to any one of embodiments 29 to 36, wherein the cells that express the first fluorescent marker, and optionally the second fluorescent marker, are isolated by fluorescence-activated cell sorting (FACS).
38. The method according to any one of embodiments 29 to 37, wherein the polynucleotides encoding the HLA alleles, and optionally the TCRs, are sequenced by Sanger sequencing.
39. The method according to any one of embodiments 29 to 38, wherein the polynucleotides encoding the HLA alleles, and optionally the TCRs, are sequenced by deep sequencing.
40. The method according to embodiment 39, wherein HLA molecules are identified as a target of an alloreactive TCR, and/or wherein TCRs are identified as alloreactive TCRs by read enrichment analysis of the deep sequencing results.

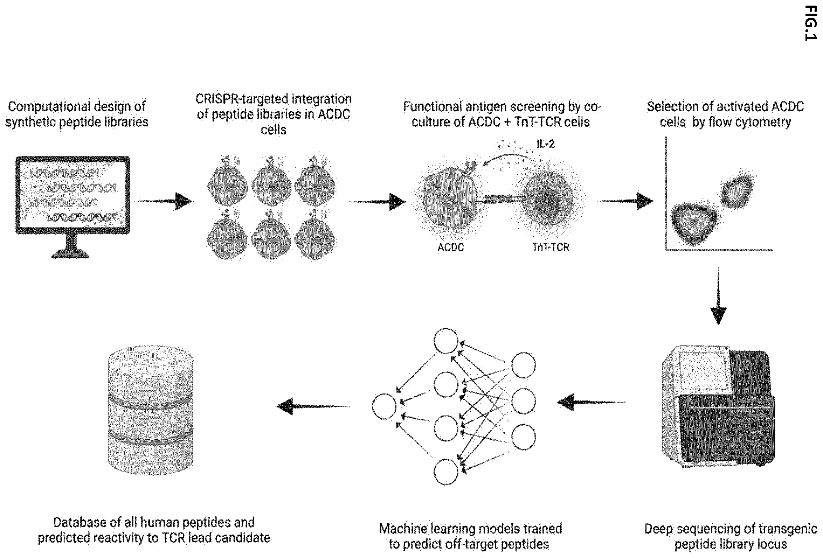

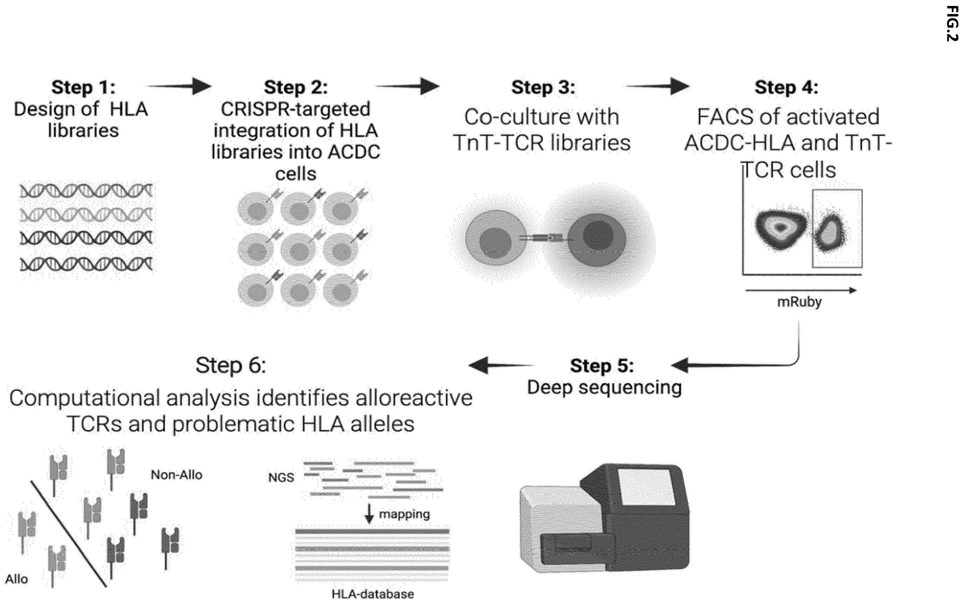

Herein, the inventors present a technology platform and data generation system enabling the molecular engineering and functional high-throughput screening of genomically-encoded candidate T cell antigen libraries and HLA allele libraries. This process, which the inventor's term TCR-Safe, combines mammalian cellular display, CRISPR-targeted mutagenesis, functional screening, deep sequencing and advanced computational methods for high-throughput profiling of cross-reactivity (**Fig. 1**) and alloreactivity (**Fig. 2**) of therapeutic TCR candidates. A core component of TCR-Safe is the Antigen presenting Cell detecting secreted Cytokine (ACDC) cell line. ACDC cells are a derivative of HEK293 cells generated by CRISPR-Cas9 genome editing in order to facilitate the display of both TCR antigen and HLA transgenes from defined genomic loci. TCR-Safe has been designed to be compatible and dependent on the previously developed TnT platform, a CRISPR-engineered human T cell line enabling functional display and selection of TCR mutagenesis libraries at high-throughput (WO 2021/074249). As such, ACDC cells harbour a fluorescent reporter of IL-2 signalling, which enables them to sense IL-2 secreted from TnT cells following antigen-specific activation. Due to its high-throughput nature (pooled screening) and use of genomically-encoded candidate T cell antigen libraries, TCR-Safe provides several advantages over traditional safety screening strategies that require substantial time and monetary resources. Furthermore, functional screening and the use of CRISPR-targeted integration of genomically-encoded antigen libraries with homogeneous expression from defined loci provide advantages over alternative approaches relying on binding affinity and retro/lentiviral transduction, respectively. By functionally screening multiple types of genomically-encoded antigen libraries, such as positional scanning libraries and combinatorial libraries of higher complexity, TCR-Safe enables not only the direct profiling of TCR specificity but also the generation of large datasets for enabling the training of machine learning models that can perform in silico predictions of TCR cross-reactivity across the human proteome. Finally, pooled screening of over 200 HLA class I alleles, aims to discard alloreactive TCRs during the engineering process itself to address another important bottleneck in TCR safety screening. Thus, TCR-Safe is a unique tool for the high-throughput safety screening of therapeutic TCRs that has the potential to greatly accelerate the development of safe and effective TCR-based immunotherapies.

Thus, in a particular embodiment, the invention relates to a cell line wherein the endogenous class I and/or class II HLA alleles are inactivated, the cell line further comprising (a) a polynucleotide encoding a first fluorescent marker under control of at least one STAT response element, and (b) an interleukin 2 (IL-2) receptor.

That is, the invention relates to a cell line for identifying targets of T cell receptors (TCRs), herein referred to as TCR antigens or antigenic peptides. The cell line of the invention is characterized in that the endogenous class I HLA alleles, the endogenous class II HLA alleles, or both the endogenous class I and class II HLA alleles are inactivated in said cell line. That is, the cell line according to the invention can no longer express an endogenous MHC class I and/or MHC class II molecule. Instead, it is intended that the cell line according to the invention is complemented with a heterologous polynucleotide encoding a class I and/or class II HLA allele.

In humans, all HLA alleles are encoded on a 3 Mbp stretch within chromosome 6, p-arm at 21.3. This stretch is also referred to as the "HLA gene complex". The α-chains of MHC class I molecules are encoded by three major genes, namely HLA-A, HLA-B and HLA-C. The α-chain undergoes heterocomplex formation with a β-microglobulin, encoded by the B2M gene on chromosome 15, to form the MHC class I molecule. The α- and β-chains of MHC class II molecules are encoded by the major genes HLA-DP, HLA-DQ and HLA-DR. All HLA genes have in common that they are highly polymorphic, which means that they have many different alleles, allowing them to fine-tune the adaptive immune system.

In certain embodiments, the invention relates to a cell line, in which the endogenous class I HLA alleles are inactivated. That is, the invention relates to a cell line in which the genes HLA-A, HLA-B and HLA-C are inactivated such that they can no longer be expressed to form a functional MHC class I α-chain.

In certain embodiments, the invention relates to a cell line, in which the endogenous class II HLA alleles are inactivated. That is, the invention relates to a cell line in which the genes HLA-DP, HLA-DQ and HLA-DR are inactivated such that they can no longer be expressed to form a functional MHC class II molecule.

The skilled person is aware of various strategies to inactivate an endogenous gene in a cell. For example, the endogenous HLA gene may be fully or partially deleted or replaced with another nucleic acid. It is important to note that the term "endogenous gene" is not limited to the coding sequence of the gene but also relates to regulatory sequences, such as promoters and other regulatory sequences. Thus, an endogenous gene may be inactivated through modifications in one of more regulatory sequences. In other words, the cell line according to the invention is a cell line that is unable to express, at least under certain conditions, a functional MHC class I and/or class II molecule.

Methods for inactivating an endogenous gene include, without limitation, CRISPR/Cas-mediated genome editing, as described in Example 2 of the present invention. Due to the high degree of polymorphism in HLA genes, it may be required to design specific gRNAs for each HLA gene. However, HLA genes comprise short stretches of highly conserved DNA sequences, which may allow inactivation of more than one HLA gene with a single gRNA. For example, the inventors have shown that a sequence in exon 4 of all three class I HLA genes can be targeted in the cell line HEK293 with a single gRNA comprising the sequence CTGCGGAGATCACACTGACC (SEQ ID NO:1). However, the skilled person is capable of designing one or more gRNAs that allow inactivation of all class I and/or class 2 HLA alleles in a cell line.

The cell line according to the invention is further designed such that it expresses a detectable marker in response to extracellular interleukin-2 (IL-2), in particular IL-2 that is secreted by a T cell in close proximity of the cell line according to the invention. That is, it is required that the cell line according to the invention comprises an IL-2 receptor.

The interleukin-2 receptor (IL-2R) is a heterotrimeric protein expressed on the surface of certain cells that binds and responds to IL-2. IL-2 binds to the IL-2 receptor, which has three forms, generated by different combinations of three different proteins, often referred to as "chains": α (alpha) (also called IL-2Rα, CD25, or Tac antigen), β (beta) (also called IL-2Rβ, or CD122), and γ (gamma) (also called IL-2Rγ, γ_{c}, common gamma chain, or CD132). The α chain binds IL-2 with low affinity, the combination of β and γ together form a complex that binds IL-2 with intermediate affinity, primarily on memory T cells and NK cells; and all three receptor chains form a complex that binds IL-2 with high affinity (K_{d} ~ 10-11 M). The intermediate and high affinity receptor forms are functional and cause changes in the cell when IL-2 binds to them.

It is thus preferred that the cell line according to the invention comprises an IL-2 receptor comprising at least IL-2Rβ and IL-R2γ. More preferably, the cell line according to the invention comprises an IL-2 receptor consisting of IL-2Rα, IL-2Rβ and IL-R2γ. Any one of IL-2Rα, IL-2Rβ and/or IL-R2γ may be encoded by the endogenous genes of the cell line. However, one or more of IL-2Rα, IL-2Rβ and/or IL-R2γ may also be encoded by transgenes that have been integrated into the cell line according to the invention. Further, IL-2Rα, IL-2Rβ and/or IL-R2γ may be wild type variants or may be engineered variants, as defined in more detail below.

To link IL-2 recognition to the expression of a detectable marker, the cell line according to the invention comprises a functional JAK/STAT signalling pathway. That is, the cell line according to the invention comprises at least one nucleic acid encoding a Janus kinase (JAK) protein family member and at least one nucleic acid encoding a signal transducer and activator of transcription (STAT) protein family member. It is preferred herein that the cell line according to the invention encodes at least JAK1 and/or JAK3. However, more preferably, the cell line according to the invention encodes JAK1 and JAK3.

The cell line according to the invention is preferably JAK3 and STATS positive. The cell line according to the invention may either comprise endogenous genes encoding JAK3 and/or STATS or may comprise transgenes encoding JAK3 and/or STAT5.

The polynucleotide encoding the detectable marker is preferably under control of a STAT response element, preferably a STATS response element. Preferably, the detectable marker is a fluorescent marker. Thus, in a particular embodiment, the cell line according to the invention comprises a polynucleotide encoding a fluorescent marker under control of a STATS response element, such that the expression of the fluorescent marker can be induced by extracellular IL-2 in a JAK/STAT-dependent manner.

It is thus preferred herein that the polynucleotide encoding the fluorescent marker is functionally linked to the IL-2 receptor via the JAK/STAT signalling pathway. That is, the polynucleotide encoding the fluorescent marker preferably comprises one or more STAT response elements to enable expression of the fluorescent marker in the presence of a suitable phosphorylated STAT dimer.

In certain embodiments, the invention relates to the cell line according to the invention, wherein the cell line comprises one or more polynucleotides encoding IL-2Rβ, IL-2Rγ, JAK3 and STATS and wherein the polynucleotide encoding the fluorescent marker is transcriptionally linked to one or more STATS response elements.

In certain embodiments, the invention relates to the cell line according to the invention, wherein the cell line comprises one or more polynucleotides encoding IL-2Rα, IL-2Rβ, IL-2Rγ, JAK3 and STATS and wherein the polynucleotide encoding the fluorescent marker is transcriptionally linked to one or more STATS response elements.

In certain embodiment, the cell line may further comprise a polynucleotide encoding JAK1.

It is to be understood that the polynucleotides encoding IL2Rα, IL2Rβ, IL-2Rγ, JAK1, JAK3 and/or STATS may be the endogenous genes encoding these proteins or may be heterologous polynucleotides that have been introduced into the cell line according to the invention.

A "STAT response element" is a regulatory DNA element that can interact with a phosphorylated STAT dimer. Thus, it is preferred herein that the polynucleotide encoding the fluorescent marker is under control of a STAT-inducible promoter. In certain embodiments, the fluorescent marker according to the invention comprises one or more STATS response element. Preferably, the STATS response element can be bound by human STATS and comprises the nucleotide sequence GGTTTTCCTGGAAAGTT (SEQ ID NO:2), TTCCTGGAA, AGTTCTGAGAAAAGT (SEQ ID NO:3) or TTCTGAGAA.

The term "fluorescent marker" as used herein refers to a protein emitting light when irradiated with excitation light. The skilled person is aware of a wide range of fluorescent proteins. That is, the fluorescent protein may be, without limitation, a green fluorescent protein (GFP), a yellow fluorescent protein (YFP), a red fluorescent protein (RFP), an orange fluorescent protein (OFP), a cyan fluorescent protein (CFP), a blue fluorescent protein (BFP), or a far-red fluorescent protein. Herein, the green fluorescent protein may be enhanced green fluorescent protein (EGFP), Emerald, Superfolder GFP (sfGFP), Azami Green, TagGFP, TurboGFP, ZsGreen or T-Sapphire, the yellow fluorescent protein may be an enhanced yellow fluorescent protein (EYFP), Topaz, Venus, mCitrine, Ypet, TagYFP, PhiYFP, ZsYellow1 or mBanana, the red fluorescent protein may be mRuby, mRuby2 mApple, mStrawberry, AsRed2 or mRFP, the orange fluorescent protein may be Kusabira Orange, Kusabira Orange2, mOrange, mOrange2, dTomato, dTomato-Tandem, TagRFP, TagRFP-T, DsRed, DsRed2, DsRed-Express, DsRed-Monomer or mTangerine, the cyan fluorescent protein may be enhanced cyan fluorescent protein (ECFP), mECFP, mCerulean, CyPet AmCyan1, Midori-Ishi Cyan, TagCFP or mTFP1, the blue fluorescent protein may be enhanced blue fluorescent protein (EBFP), EBFP2, Azurite or mTagBFP, and the far red fluorescent protein may be mPlum, mCherry, dKeima-Tandem.

The term "cell line", as used herein, refers to a cell culture comprising a single cell type that can be serially propagated in culture for prolonged periods. The cell line is preferably a mammalian cell line. In certain embodiments, the cell line is a murine cell line. It is however preferred that the cell line is a human cell line. In certain embodiments, the human cell line is derived from HEK 293.

Human embryonic kidney 293 cells, also often referred to as HEK 293, HEK-293, 293 cells, or less precisely as HEK cells, are a specific immortalized cell line derived from a spontaneously miscarried or aborted fetus or human embryonic kidney cells grown in tissue culture taken from a female fetus in 1973. HEK 293 cells have been widely used in cell biology research for many years, because of their reliable growth and propensity for transfection. They are also used by the biotechnology industry to produce therapeutic proteins and viruses for gene therapy as well as safety testing for a vast array of chemicals.

In a particular embodiment, the invention relates to the cell line according to the invention, wherein the polynucleotide encoding the first fluorescent marker is under control of at least 2, 3, 4 or 5 STAT response elements.

That is, in certain embodiments, the reporter system comprising the polynucleotide encoding the fluorescent marker comprises at least 1, 2, 3, 4 or 5 STAT response elements. In certain embodiments, the reporter system comprising the polynucleotide encoding the fluorescent marker comprises 1, 2, 3, 4, 5, 6, 7, 8, 9 or 10 STAT response elements. In certain embodiments, the reporter system comprising the polynucleotide encoding the fluorescent marker comprises 5 STAT response elements. Preferably, the STAT response elements are STATS response elements.

In a particular embodiment, the invention relates to the cell line according to the invention, wherein the IL-2 receptor is an engineered IL-2 receptor, in particular wherein the engineered IL-2 receptor comprises an engineered common gamma chain.

In certain embodiments, the IL-2 receptor is a wild-type IL-2 receptor. However, the IL-2 receptor may also be an engineered IL-2 receptor. In certain embodiments, the IL-2 receptor may be engineered to be more responsive to IL-2.

In certain embodiments, the engineered IL-2 receptor may comprise one or more mutations in the common gamma chain that confers enhanced responsiveness to IL-2. In certain embodiments, the engineered common gamma chain variant is derived from a human common gamma chain (SEQ ID NO:4). In certain embodiments, the engineered common gamma chain variant is derived from a human common gamma chain (SEQ ID NO:4) and comprises one or more mutations at any one of positions Arg204, Phe205, Asn206, Pro207, Leu208, Cys209, Gly210, Ser211, Ala212, Gln213 and/or His214. In certain embodiments, the engineered common gamma chain variant is derived from a human common gamma chain (SEQ ID NO:4) and comprises mutations at positions P207 and/or H214. In certain embodiments, the engineered common gamma chain variant is derived from a human common gamma chain (SEQ ID NO:4) and comp comprises mutations P207K and/or H214K.

However, the engineered IL-2 receptor may comprise other or additional mutations in anyone of the IL-2 receptor subunits. Preferably, the additional mutations in the subunits of the engineered IL-2 receptor may confer enhanced responsiveness to IL-2.

It is preferred that the IL-2 receptor is of the same origin as the cell line according to the invention. That is, when the cell line is a human cell line, such as a HEK 293 cell line, the IL-2 receptor is preferably a human IL-2 receptor.

In a particular embodiment, the invention relates to the cell line according to the invention, wherein the cell line further comprises a polynucleotide encoding a sequence-specific endonuclease, in particular wherein the sequence-specific endonuclease is a CRISPR-associated (Cas) protein, in particular wherein the CRISPR-associated (Cas) protein is Cas9.

Cell lines encoding a CRISPR-associated protein have the advantage that it is no longer necessary to introduce a purified CRISPR-associated protein together with the tracrRNA and the crRNA in every genome editing step.

The polynucleotide encoding the CRISPR-associated protein may be introduced into the cell line according to the invention by any method known in the art. For example, the polynucleotide encoding the CRISPR-associated protein may be integrated into the genome of the cell line according to the invention by CRISPR/Cas-mediated genome editing as known in the art. Alternatively, the polynucleotide encoding the CRISPR-associated protein may be integrated into the genome of the cell line according to the invention by viral transduction.

The term "*CRISPR-associated protein"* refers to RNA-guided endonucleases that are part of a CRISPR (Clustered Regularly Interspaced Short Palindromic Repeats) system (and their homologs, variants, fragments or derivatives), which is used by prokaryotes to confer adaptive immunity against foreign DNA elements. CRISPR-associated proteins include, without limitation, Cas9, Cpf1 (Cas12), C2c1, C2c3, C2c2, Cas13, CasX and CasY.

As used herein, the term "*CRISPR-associated protein*" includes wild-type proteins as well as homologs, variants, fragments and derivatives thereof. Therefore, when referring to artificial nucleic acid molecules encoding Cas9, Cpf1 (Cas12), C2c1, C2c3, and C2c2, Cas13, CasX and CasY, said artificial nucleic acid molecules may encode the respective wild-type proteins, or homologs, variants, fragments and derivatives thereof. In certain embodiments, the CRISPR-associated protein is Cas9. In certain embodiments, Cas9 is encoded by a polynucleotide that is codon optimized for use in mammalian or, more preferably human, cells. Codon optimized polynucleotides encoding Cas9 are known in the art.

It is important to note that the presence of a polynucleotide encoding a CRISPR-associated protein is not an essential feature of the cell line according to the invention. That is, the cell line according to the invention may have been obtained without using the CRISPR/Cas method or, where the cell line according to the invention has been obtained using the CRISPR/Cas method, the polynucleotide encoding the CRISPR-associated protein may have been removed from the genome of the cell line subsequently.

In a particular embodiment, the invention relates to the cell line according to the invention, wherein the cell line further comprises a heterologous polynucleotide encoding an HLA allele.

That is, the cell line according to the invention may be complemented with a heterologous polynucleotide encoding an HLA allele. In a particular embodiment, the invention relates to the cell line according to the invention, wherein the endogenous class I HLA alleles are inactivated in said cell line and wherein the cell line comprises a heterologous polynucleotide encoding a class I HLA allele.

That is, the invention relates to a cell line according to the invention in which the endogenous class I HLA alleles (HLA-A, HLA-B and HLA-C) have been inactivated and wherein the cell line has been complemented with a heterologous class I HLA allele. Such a cell line may be pulsed with an antigenic peptide and used as antigen-presenting cell for the discovery of TCRs. Furthermore, such cells may be contacted with T cells in the absence of an antigenic peptide to assess the alloreactivity of TCRs, as described in more detail below.

In a preferred embodiment, the cell line according to the invention is a cell line in which the class I HLA alleles encoded by the genes HLA-A, HLA-B and HLA-C are inactivated as described herein and wherein the cell line further comprises a heterologous polynucleotide encoding a class I HLA allele. In another embodiment, the cell line according to the invention is a cell line in which the class II HLA alleles encoded by the genes HLA-DP, HLA-DQ and HLA-DR are inactivated and wherein the cell line further comprises a heterologous polynucleotide encoding a class II HLA allele. In yet another embodiment, the cell line according to the invention is a cell line in which the class I HLA alleles encoded by the genes HLA-A, HLA-B and HLA-C and the class II HLA alleles encoded by the genes HLA-DP, HLA-DQ and HLA-DR are inactivated and wherein the cell line further comprises a heterologous polynucleotide encoding a class I and/or a class II HLA allele. Preferably, the heterologous HLA allele is of the same origin as the cell line.

The heterologous polynucleotide encoding the HLA allele may be integrated into the cell line according to the invention by any method known in the art. For example, the heterologous polynucleotide encoding the HLA allele may be integrated into the genome of the cell line by viral transduction. Suitable viral vectors and methods are known to the person skilled in the art. However, it is preferred herein that the heterologous polynucleotide encoding the HLA allele is integrated into the genome of the cell line according to the invention in a site-directed manner, preferably by CRISPR-mediated homology directed repair, as disclosed herein in Example 3.

Successful integration of the heterologous polynucleotide encoding the HLA allele may be verified by flow cytometry. In particular, anti-HLA antibodies are known in the art and may be used in separating cells expressing a heterologous polynucleotide encoding the HLA allele from HLA-negative cells, i.e. cells according to the invention in which the endogenous HLA alleles have been inactivated.

The HLA allele may be any class I or class II HLA allele. For example, the class I or class II allele may be, without any one of the HLA alleles comprised in the IPD-IMGT/HLA Database (https://www.ebi.ac.uk/ipd/imgt/hla/).

A cell line comprising a heterologous HLA allele may be pulsed with a peptide as known in the art. For example, 1 × 10⁶ cells of the cell line according to the invention may be washed with serum-free RPMI-1640 and resuspended in 1 mL of serum-free RPMI-1640 containing 5 × 10⁻⁵ molar to 5 × 10⁻¹² molar peptide. Peptide pulse may be allowed for 90 min at 37 °C, 5% CO₂. The cells may then be washed and resuspended at 10⁶ cells per mL in complete media containing 10% FBS. Peptide pulse of lower amounts of cells may be performed by reducing the amounts of resuspension volume proportionally and using 96-well, 48-well, 24-well, 6-well microplates or 10 cm tissue culture dishes.

In a particular embodiment, the invention relates to the cell line according to the invention, wherein the endogenous gene encoding beta-2 microglobulin is inactivated in said cell line.

Inactivating the endogenous gene encoding beta-2 microglobulin (B2M) in the cell line according to the invention results in a cell line that can no longer express a functional MHC class I molecule on the cell surface. Such a cell line may be used for the identification of MHC class I antigenic peptides, for example by complementing the cell line with a heterologous polynucleotide encoding a beta-2 microglobulin fused to an antigenic peptide, as described in more detail herein below.

The endogenous gene encoding beta-2-microglobulin may be inactivated by any method known in the art. For example, the B2M gene may be inactivated using the CRISPR/Cas method as described in Example 5.

Thus, in certain embodiments, the cell line according to the invention may be a cell line in which at least the endogenous HLA-A, HLA-B, HLA-C and B2M genes are inactivated. Preferably, said cell line further comprises a heterologous polynucleotide encoding a class I HLA allele.

In an alternative embodiment, the invention further relates to a cell line wherein the endogenous gene encoding beta-2-microglobulin is inactivated, but wherein not all (or even none) of the endogenous genes encoding the HLA alleles are inactivated. Thus, in an alternative embodiment, the invention relates to a cell line wherein the endogenous gene encoding beta-2-microglobulin is inactivated, the cell line further comprising (a) a polynucleotide encoding a first fluorescent marker under control of at least one STAT response element, and (b) an interleukin 2 (IL-2) receptor.

A cell line wherein the endogenous B2M gene is inactivated may be used for the identification of MHC class I antigenic peptides. In these embodiments, expressing a heterologous polynucleotide encoding a beta-2 microglobulin and an antigenic peptide can result in the formation of an MHC-peptide complex with an endogenous MHC class I α-chain.

In a particular embodiment, the invention relates to the cell line according to the invention, wherein the cell line further comprises a heterologous polynucleotide encoding a beta-2 microglobulin.

That is, the cell line according to the invention may be complemented with a heterologous polynucleotide encoding a beta-2 microglobulin. The heterologous polynucleotide encoding the beta-2 microglobulin may be introduced into the cell line according to the invention by any method known in the art. Preferably, the heterologous polynucleotide encoding the beta-2 microglobulin is introduced into the genome of the cell line according to the invention. More preferably, the heterologous polynucleotide encoding the beta-2 microglobulin is introduced into the genome of the cell line according to the invention by CRISPR-mediated homology directed repair. It is preferred herein that the beta-2 microglobulin is from the same origin as the cell line.

The heterologous polynucleotide encoding the beta-2 microglobulin may be introduced into any one of the cell lines defined above. That is, the heterologous polynucleotide encoding the beta-2 microglobulin may be introduced into a cell line with or without an inactivated endogenous B2M gene. However, it is preferred that the heterologous polynucleotide encoding the beta-2 microglobulin is introduced into a cell line having an inactivated endogenous B2M gene such that the inactivated endogenous B2M gene is complemented in said cell line.

Successful integration of the heterologous polynucleotide encoding the beta-2 microglobulin may be verified by flow cytometry. In particular, anti-B2M antibodies are known in the art that allow separating cells that express a heterologous polynucleotide encoding the B2M from B2M-negative cells, i.e. cells in which the endogenous B2M gene has been inactivated.

In a particular embodiment, the invention relates to the cell line according to the invention, wherein the cell line further comprises a polynucleotide encoding a peptide, preferably wherein the polynucleotide encoding the beta-2 microglobulin and the polynucleotide encoding the peptide are transcriptionally fused.

That is, the heterologous polynucleotide encoding the beta-2 microglobulin may further be fused to a polynucleotide encoding a peptide. It is preferred herein that the polynucleotide encoding the beta-2 microglobulin and the polynucleotide encoding the peptide are transcriptionally fused. That is, the two polynucleotides are fused in a way such that they are transcribed into a single mRNA.

The peptide is preferably an antigenic peptide, in particular an antigenic peptide that can be displayed by an MHC class I molecule. Thus, in a particular embodiment, the invention relates to the cell line according to the invention, wherein the peptide is an MHC class I peptide.

An "MHC class I peptide" is a peptide that can be displayed by an MHC class I molecule. MHC class I peptides typically have a length of 8-12 amino acid residues. However, the peptide that is fused to the beta-2 microglobulin may be a longer peptide that comprises an MHC class I peptide. Thus, in certain embodiments, the peptide that is fused to the beta-2 microglobulin preferably has a length of 8-30 amino acids, 8-25 amino acids, 8-20 amino acids, 8-15 amino acids, 8-12 amino acids or 8-10 amino acids.

The polynucleotide encoding the peptide is preferably fused to the 3' end of the polynucleotide encoding the beta-2 microglobulin. In certain embodiments, the polynucleotide encoding the peptide may be fused directly to the beta-2 microglobulin molecule. That is, the polynucleotide encoding the peptide and the polynucleotide encoding the beta-2 microglobulin molecule may be transcriptionally and translationally fused.

It is however preferred that the polynucleotide encoding the peptide is fused to the beta-2 microglobulin molecule via a linker. Thus, in a particular embodiment, the invention relates to the cell line according to the invention, wherein the polynucleotide encoding the beta-2 microglobulin and the polynucleotide encoding the peptide are fused via a linker.

In certain embodiments, the linker may be a flexible peptide linker that provides the spatial freedom that is required for the peptide to be presented by the MHC class I molecule. That is, the polynucleotide encoding the peptide and the polynucleotide encoding the beta-2 microglobulin molecule may be transcriptionally and translationally fused via a polynucleotide encoding a flexible peptide linker.

In a preferred embodiment, the linker may encode one or more elements that allow the peptide to be separated from the beta-2 microglobulin either during translation or subsequent to translation. That is, the linker may preferably encode a self-cleaving peptide and/or a protease-specific cleavage site.

Thus, in a particular embodiment, the invention relates to the cell line according to the invention, wherein the linker encodes a protease-specific cleavage site and/or a self-cleaving peptide.

That is, in certain embodiments, the heterologous polynucleotide may comprise a polynucleotide encoding a beta-2 microglobulin and a polynucleotide encoding a peptide, wherein the two polynucleotides are connected by a linker encoding a protease-specific cleavage site. That is, the resulting polypeptide may be cleaved subsequent to its translation by a protease that separates the peptide from the beta-2 microglobulin. The "protease-specific cleavage site" may be any sequence motif that is recognized and cleaved by a protease, preferably by a mammalian intracellular protease. In certain embodiments, the "protease-specific cleavage site" may be any sequence motif that is recognized and cleaved by a human protease, preferably an intracellular human protease. In certain embodiments, the "protease-specific cleavage site" is a Furin cleavage site comprising the sequence motif Arg-X-(Arg/Lys) -Arg.

In certain embodiments, the heterologous polynucleotide may comprise a polynucleotide encoding a beta-2 microglobulin and a polynucleotide encoding a peptide, wherein the two polynucleotides are connected by a linker encoding a self-cleaving peptide. As used herein, the term "self-cleaving peptide" means a peptide sequence having a cleavage activity that occurs between two amino acid residues in the peptide sequence itself. Examples of the self-cleaving peptide include a 2A peptide and a 2A-like peptide. For example, in 2A or 2A-like peptides, cleavage occurs between glycine and proline residues on these peptides. This is caused by the "ribosome skipping mechanism", which prevents the formation of normal peptide bonds between glycine residues and proline residues during translation, and does not affect downstream translation. The ribosome skip mechanism is known in the art and is used for the expression of multiple proteins encoded by a single molecule of messenger RNA (mRNA). Various self-cleaving peptides are known in the art and include, without limitation, T2A (EGRGSLLTCGDVEENPGP (SEQ ID NO:5)), P2A (ATNFSLLKQAGDVEENPGP (SEQ ID NO:6)), E2A (QCTNYALLKLAGDVESNPGP (SEQ ID NO:7)) and F2A (VKQTLNFDLLKLAGDVESNPGP (SEQ ID NO:8)).

In certain embodiments, the heterologous polynucleotide may comprise a polynucleotide encoding a beta-2 microglobulin and a polynucleotide encoding a peptide, wherein the two polynucleotides are connected by a linker encoding a protease-specific cleavage site and a self-cleaving peptide. In certain embodiments, the heterologous polynucleotide may comprise a polynucleotide encoding a beta-2 microglobulin and a polynucleotide encoding a peptide, wherein the two polynucleotides are connected by a linker encoding a Furin cleavage site and a self-cleaving peptide. In certain embodiments, the heterologous polynucleotide may comprise a polynucleotide encoding a beta-2 microglobulin and a polynucleotide encoding a peptide, wherein the two polynucleotides are connected by a linker encoding a Furin cleavage site and a P2A motif.

In certain embodiments, the peptide may be further linked to a signal peptide to enable secretion of the peptide to the extracellular space, where it can then be displayed by an MHC molecule. Thus, in a particular embodiment, the invention relates to the cell line according to the invention, wherein the peptide further comprises a signal peptide.

That is, the peptide may be fused to any type of signal peptide that allows sufficient secretion of the peptide to the extracellular space. Preferably, the signal peptide allows secretion of the peptide from mammalian cells, more preferably from human cells. Various signal peptides are known in the art and can be used by the person skilled in the art to achieve secretion of the peptide. In one non-limiting embodiment, the signal peptide is the human IL-2 secretory signal peptide (MYRMQLLSCIALSLALVTNS (SEQ ID NO:9)).

In a preferred embodiment, the heterologous polypeptide encoding the beta-2 microglobulin and the peptide has the following structure: 5'-B2M - protease-specific cleavage site - self-cleaving peptide - signal peptide - peptide -3'. In a more preferred embodiment, the heterologous polypeptide encoding the beta-2 microglobulin and the peptide has the following structure: 5'-B2M - Furin cleavage site - self-cleaving peptide - signal peptide - peptide -3'. In a most preferred embodiment, the heterologous polypeptide encoding the beta-2 microglobulin and the peptide has the following structure: 5'-B2M - Furin cleavage site - 2A self-cleaving peptide - signal peptide - peptide -3'. As mentioned above, the peptide is preferably an MHC class I peptide or a potential MHC class I peptide, i.e. a peptide having a length of 8-12 amino acid residues.

Cells that efficiently display an antigenic peptide, irrespective whether the peptide was pulsed on the cell line according to the invention or was genetically encoded in the cell line according to the invention, may be detected with soluble TCRs as known in the art. For that, cells may be harvested from culture, pelleted by centrifugation and culture media may be removed with a pipette. Cells may then be placed on ice and washed once in ice-cold flow cytometry buffer (PBS, 2% FBS, 2 mM EDTA). Next, cells may be resuspended in solutions containing fluorescently labelled sTCRs at concentrations ranging from 1 pM to 50 µM and incubated on ice for 20 minutes. Cells may then be washed twice with flow cytometry buffer and analysed by flow cytometry to detect surface bound fluorescently labelled sTCRs.

The present invention further relates to methods for generating any of the cell lines defined herein above.

In a particular embodiment, the invention relates to a method for identifying potential off-targets of a T cell receptor (TCR), the method comprising the steps of: (a) providing a plurality of cells according to the invention, wherein at least two cells comprised in the plurality of cells encode a different peptide variant that has been obtained by mutagenesis of a known antigenic peptide; (b) contacting the plurality of cells of step (a) with a plurality of T cells encoding a TCR that is specific for said known antigenic peptide; (c) isolating cells that express the first fluorescent marker; (d) sequencing the polynucleotides encoding the peptide variants in the cells that have been isolated in step (c); and (e) identifying potential off-targets of the TCR of interest based on the sequencing results that have been obtained in step (d).

The cell line according to the invention may be used for identifying potential off-targets of a known TCR or, more specifically, for determining the cross-reactivity of a known TCR. For that, it is required that both the TCR and the antigenic peptide to which the TCR binds are known. The "known TCR" can be a naturally occurring TCR or a TCR that has been obtained by genetic engineering. A TCR is a "known TCR" if the amino acid sequence of said TCR is known and if at least one antigenic peptide is known that is recognized by said TCR. An antigenic peptide is a "known antigenic peptide" if the amino acid sequence of the antigenic peptide is known and if the peptide has been confirmed to be recognized by a known TCR.

To identify potential off-targets of the known TCR, a DNA library encoding a plurality of variants of the known antigenic peptide may be screened in the cell line according to the invention.

The library may be obtained with any method known in the art. For example, the library may be obtained by random mutagenesis of a polynucleotide encoding the known antigenic peptide, for example by error prone PCR. Alternatively, the library may be obtained by site-saturation mutagenesis of the polynucleotide encoding the known antigenic peptide at one or more positions. Thus, in a particular embodiment, the invention relates to the method according to the invention, wherein the peptide variants have been obtained by site-directed mutagenesis of the known antigenic peptide, in particular by site-directed saturation mutagenesis of the known antigenic peptide.

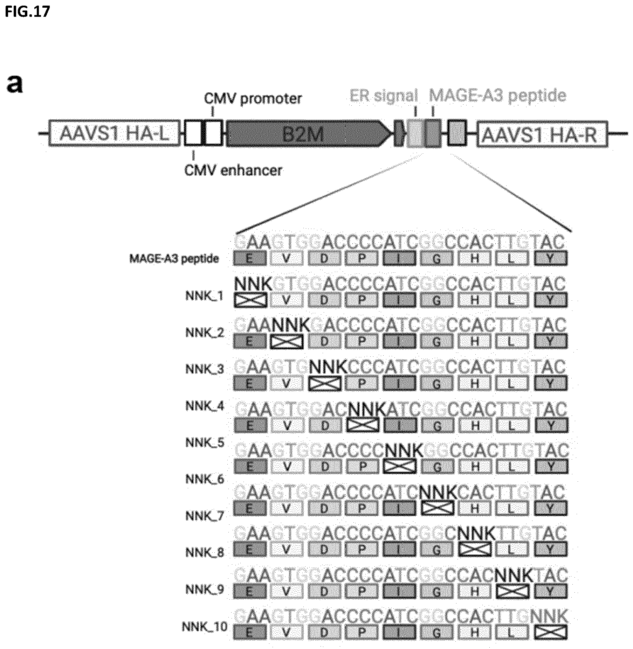

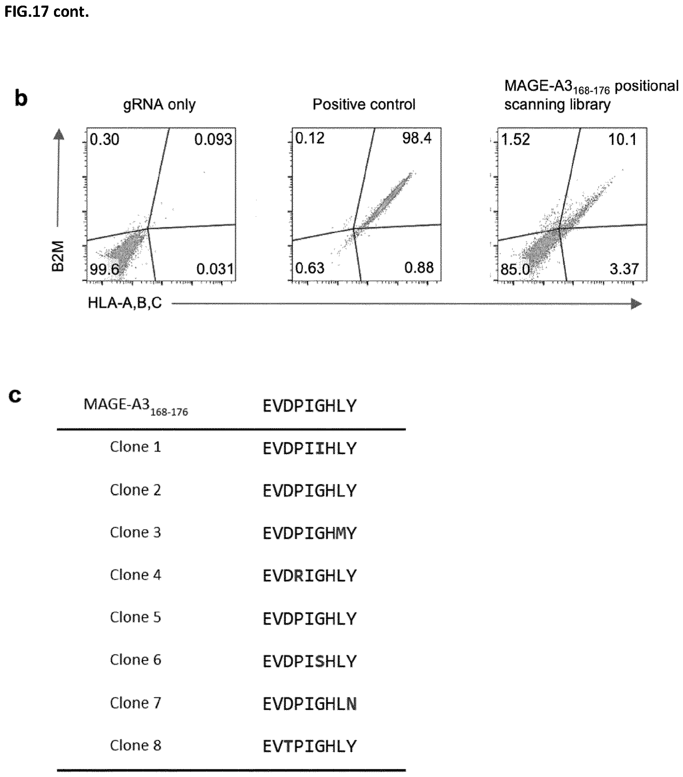

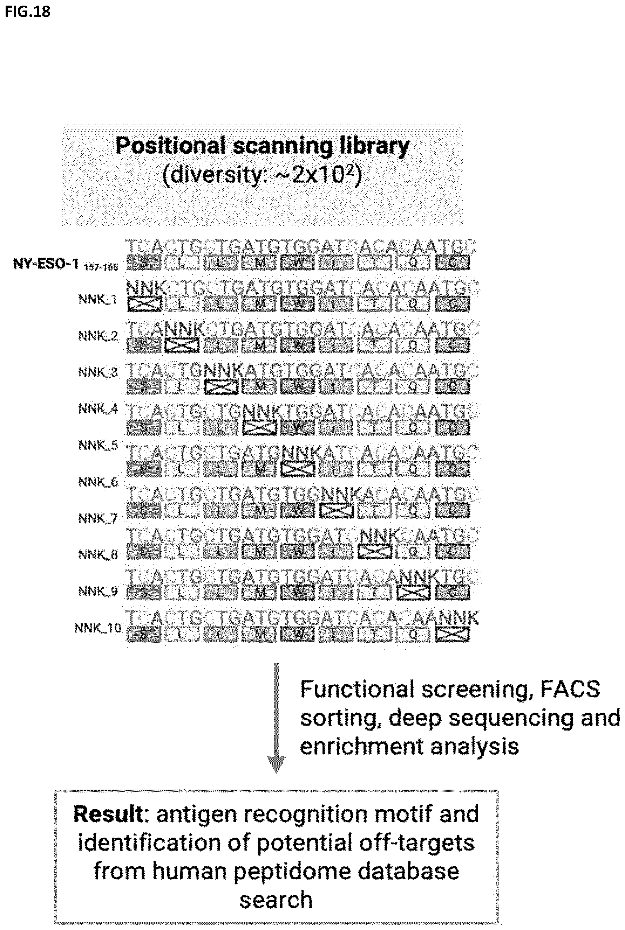

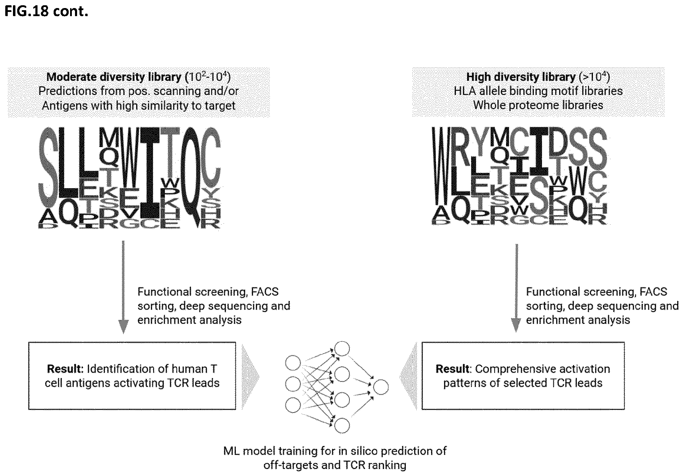

Preferably, the library is obtained by positional scanning mutagenesis. The term "positional scanning" refers to a technology that systematically substitutes the amino acid residues at each position within a certain peptide with other amino acids. Preferably, substitution of the amino acid is achieved by introducing a degenerated NNK codon, which encodes all 20 canonical amino acids, as exemplified for two antigenic peptides in FIGs. 17 and 18. Thus, positional scanning mutagenesis is a specific type of site-saturation mutagenesis.

It is preferred herein that the polynucleotides encoding the peptide variants are transcriptionally linked to a polynucleotide encoding a beta-2 microglobulin as disclosed herein above. It is further preferred that the polynucleotides encoding the beta-2 microglobulin and the polynucleotides encoding the peptide variants are linked by one of the linkers disclosed herein above, in particular linkers encoding a protease-specific cleavage site, a self-cleaving peptide and a signal peptide.

The DNA library may be prepared as disclosed herein. That is, site-specific mutagenesis may be used for library construction. The primers may be designed and purchased as (i) a pool of reverse primer containing tiled NNK degenerate codons across the sequence encoding the target T cell antigen, and (ii) the forward primer with 5' phosphorylation may be designed such that the 5' ends of the two primers anneal back-to-back. 30 nmol of each reverse primer and 300 nmol of forward primer may be mixed and subjected to PCR amplification on a plasmid encoding the B2M-antigenic peptide fusion. T4 ligase may be used for circularization of the PCR product for 2 h at RT. Ligation mixes may be transformed into NEB5α bacteria according to the manufacturer's instructions. Each transformation mix may be plated on ampicillin LB agar in Square Bioassay dishes. 1/100 and 1/1000 dilutions of the transformations may also be prepared and plated on ampicillin plates to determine the number of transformants the following day. Colonies from the Square Bioassay plates may be collected by adding 15 mL of LB media to each plate and resuspending. Colonies from each library may be pooled together in a single tube and centrifuged at 4800 g for 10 min. The supernatant may be discarded and the plasmid DNA may be isolated following the Midiprep protocol.

The peptide variants encoded in the DNA library may have a length of 8-30, 8-25, 8-20, 8-15, 8-12 or 8-10 amino acids. However, it is preferred that the peptide variants encoded in the DNA library have the same length as the known antigenic peptide and that each peptide variant differs from the known antigenic peptide in one or more amino acid residues.

In a first step, a plurality of cells may be obtained by introducing the DNA library into the cell line according to the invention. It is preferred that at least two cells that are obtained by introducing the DNA library into the cell line according to the invention encode a different peptide variant, i.e. a different variant of the known antigenic peptide. However, more preferably, at least 5, 10, 20, 50, 100, 200, 300, 500 or 1000 cells in the plurality of cells encode a different variant of the known antigenic peptide. Thus, in a particular embodiment, the invention relates to the method according to the invention, wherein the plurality of cells encode at least 5, 10, 20, 50, 100, 200, 300, 500 or 1000 different peptide variants that have been obtained by mutagenesis of a known antigenic peptide.

The cell line may be any cell line disclosed herein, in particular any cell line comprising a functional IL-2 receptor and a polynucleotide encoding a fluorescent marker under control of the JAK/STAT pathway as disclosed herein. Preferably, the cell line is a cell line in which the endogenous class I HLA genes and, even more preferably, also the endogenous B2M gene, have been inactivated and wherein the cell line has been complemented with a heterologous polynucleotide encoding a class I HLA allele. In embodiments where the endogenous B2M gene has been inactivated, the cell line further has to be complemented with a heterologous polynucleotide encoding B2M, which is preferably introduced into the cell line as part of the DNA library.

The DNA library may be introduced into the cell line according to the invention by any suitable method known in the art. That is, the skilled person is aware of methods to introduce a DNA molecule into a cell line. For example, the DNA library may be introduced into the cell line according to the invention by transfection, as disclosed in the appended examples.

The term "transfection" as used herein refers to the introduction of foreign DNA into eukaryotic cells. Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics. Preferably, the DNA library is transfected into the cell line according to the invention by electroporation, as disclosed in the appended examples.

In a second step, the plurality of cells that has been obtained by introducing the DNA library into the cell line according to the invention may be contacted with a plurality of T cells. The T cells in the plurality of T cells are preferably T cells that are specific for the known antigenic peptide based on which the DNA library has been generated. That is, it is preferred that at least 70%, at least 75%, at least 80%, at least 85%, at least 90%, at least 95% or at least 99% of the T cells in the population of T cells comprise a T cell receptor that is specific for the known antigenic peptide based on which the DNA library has been generated.

The plurality of cells encoding the antigenic peptide variants and the T cells may contacted at any suitable ratio. In particular, cells encoding the antigenic peptide variants may be contacted with T cells at a ratio ranging from 0.1 to 10. Furthermore, cells encoding the antigenic peptide variants may be contacted with T cells in a volume ranging from 0.15 mL to 15 mL and with a total cell number ranging from 1×10³ to 1×10⁷ cells per cell culture.

Further, the plurality of cells encoding the antigenic peptide variants and the T cells may contacted under suitable conditions. In particular, cells are preferably cultured in complete media supplemented with 1 µg/mL anti-CD28 antibody (i.e. BioLegend, #302933). Cells may be cultured at at 37 °C and 5% CO₂. Cells are preferably cultured overnight, and cells are subsequently analyzed for expression of a fluorescent marker or an activation marker on the surface of T cells, such as CD69.

The skilled person is capable of identifying the optimal conditions for contacting cells encoding the antigenic peptide variants with T cells such that sufficient expression of the fluorescent markers, or other activation markers can be achieved. It is important to note that the conditions disclosed herein may be applied to any one of the methods according to the invention.

The T cell may be any suitable T cell that can recognize the known antigenic peptide based on which the library has been generated. That is, the T cell is preferably a cell that comprises a T cell receptor that is specific for the known antigenic peptide and further comprises a suitable co-receptor, such as CD4 or CD8, such that the known antigenic peptide can be recognized when presented by an MHC class I or II molecule. Furthermore, the T cell is required to secrete IL-2 when activated by an antigenic peptide in a TCR-dependent manner. In a preferred embodiment, the T cell is derived from the TnT cell line as disclosed in WO 2021/074249, which is fully incorporated herein by reference.

In a next step, cells presenting peptide variants that are recognized by the known TCR are isolated based on the expression of the fluorescent marker. Recognition of a peptide variant by a T cell results in the activation of the T cell and thus in the secretion of IL-2 by the T cell. The secreted IL-2 can then be recognized by the IL-2 receptor of the antigen-presenting cell according to the invention and induce the expression of the fluorescent marker in a JAK/STAT-dependent manner. Thus, presentation of a peptide variant that can result in the activation of the T cell will ultimately result in the expression of the fluorescent marker in the cell line according to the invention.

Contacting a plurality of cells presenting different peptide variants with a plurality of T cells expressing a known TCR will result in expression of a fluorescent marker in all cells that present a TCR-recognizable peptide variant. These cells can then be isolated based on the expression level of the fluorescent marker. Fluorescent cells can readily be separated from non-fluorescent cells, for example by fluorescence-activated cell sorting (FACS). Methods and devices for separating fluorescent cells from non-fluorescent cells are well known in the art. Furthermore, the skilled person is capable of isolating sub-populations of fluorescent cells, i.e. a highly fluorescent sub-population of cells. Thus, in a particular embodiment, the invention relates to the method according to the invention, wherein the cells that express the first fluorescent marker are isolated by fluorescence-activated cell sorting (FACS).

The steps of contacting cells that present the peptide variants with T cells may be repeated several times to reduce the risk of false-positives. That is, the cells that present the peptide variants may be contacted with the T cells for 2, 3, 4, 5 or more times, wherein the fluorescent cells are isolated after each contacting step and contacted with a fresh batch of T cells.

After the final isolation step, polynucleotides encoding the peptide variants are isolated from the cells and sequenced, preferably including a step of amplifying a region of interest before the sequencing step. In certain embodiments, a single cell may be isolated from the population of fluorescent cells and subjected to Sanger sequencing as known in the art to obtain a nucleotide sequence encoding a peptide variant that has the potential to activate the known TCR. Thus, in a particular embodiment, the invention relates to the method according to the invention, wherein the polynucleotides encoding the peptide variants are sequenced by Sanger sequencing.

However, it is preferred that that the entire population of fluorescent cells is subjected to deep sequencing. Thus, in a particular embodiment, the invention relates to the method according to the invention, wherein the polynucleotides encoding the peptide variants are sequenced by deep sequencing.

The term *"deep sequencing"* as used herein generally refers to next- or higher-generation sequencing known to a skilled artisan. In particular, the term "*deep sequencing*" as used herein indicates that the total number of reads is many times larger than the length of the sequence under study. The method according to the invention preferably comprises a step of positional scanning involving a combination of multi-site saturation mutagenesis, phenotypic screening (preferably by FACS) and deep sequencing.

Deep sequencing results in a large number of reads which may be assigned to specific peptide variants, preferably by using bioinformatic methods known in the art. Peptide variants that can activate the known TCR can then be identified by analysing the abundance of reads encoding these peptide variants.

Preferably, peptide variants that are recognized by a known TCR are identified by read enrichment analysis. Thus, in a particular embodiment, the invention relates to the method according to the invention, wherein potential off-targets are identified by read enrichment analysis of the deep sequencing results.

For that, it is preferred that at least two populations of cells are subjected to deep sequencing. The first population is the fluorescent population of cells that has been obtained after the final isolation step. That is, the first population of cells is a population in which TCR-recognizable peptide variants have been enriched. The second population of cells is a non-fluorescent population, preferably from the same batch from which the fluorescent population has been isolated.

By comparing the deep sequencing results of the two populations it is possible to determine the enrichment factor of each peptide variant that is comprised in the library. Peptide variants that are enriched in the fluorescent population can then be identified as peptide variants that can activate the known TCR, and consequently as potential off-targets of the known TCR.

Accordingly, any peptide variant in the fluorescent population, irrespective of whether it has been identified by Sanger sequencing of deep sequencing, can be considered a potential off-target of the known TCR.

In a particular embodiment, the invention relates to the method according to the invention, the method comprising an additional step of querying a potential off-target of a TCR that has been identified in step (e) against a protein database.

The method according to the invention can be used for identifying potential off-targets of known TCRs. The identified potential off-targets may then, in a next step, be queried against a protein database to identify whether the potential off-target may be an actual off-target of the known TCR. In particular, potential off-targets may be queried against a protein database that comprises protein sequences from the same organism from which the known TCR has been derived. Preferably, the method according to the invention is used for identifying potential off-targets of human TCRs. Thus, the identified potential off-targets may be queried against a human protein database. Suitable protein databases against which the potential off-target may be queried are known to the person skilled in the art.

Preferably, the method of the invention is used to determine the cross-reactivity of engineered TCRs. Engineering TCRs for a specific antigen bears the risk of introducing unwanted reactivity to off-targets which may cause severe or even fatal side-effects when these engineered TCRs are administered to a patient. Thus, the method according to the invention may be used to identify and eliminate cross-reactive engineered TCRs at an early point of development. At the same time, engineered TCRs with no substantial cross-reactivity may be identified and further developed for the safe use in patients.

In a particular embodiment, the invention relates to the method for identifying a target of a T cell receptor (TCR) of interest, the method comprising the steps of: (a) providing a plurality of cells according to the invention, wherein at least two cells comprised in the plurality of cells encode a different peptide candidate; (b) contacting the plurality of cells of step (a) with a plurality of T cells encoding a TCR of interest; (c) isolating cells that express the first fluorescent marker; (d) sequencing the nucleic acids encoding the peptide candidates in the cells that have been isolated in step (c); and (e) identifying a target of the TCR of interest based on the sequencing results that have been obtained in step (d).

The cell line according to the invention may not only be used for identifying potential off-targets of a known TCR, but also for identifying targets of a TCR of interest. A "TCR of interest" is a TCR with a known or accessible amino acid sequence. An amino acid sequence of a "TCR of interest" is accessible if an isolated cell is available from which the amino acid sequence of the TCR can be derived with methods known in the art. The "TCR of interest" may be a TCR for which no antigenic peptide has been identified.

Since the antigenic peptide that is recognized by the TCR of interest is not known, the potential sequence space is very large (e.g. 20¹⁰ for 10-mer peptides). The method of the invention has the advantage that highly diverse and genetically encoded peptide libraries can be generated and tested in the cell line according to the invention. Thus, the method of the invention enables the screening of large numbers of peptide candidates without the need for costly synthesis of these peptides.

The DNA library encoding the peptide candidates may be generated with any method known in the art. In certain embodiments, the genetic information of the peptide candidates encoded in the library may be derived from databases. In certain embodiments, the DNA library encoding the peptide candidates may be designed based on information that has been retrieved from publicly available peptide databases. In other embodiments, the DNA library encoding the peptide candidates may be designed based on information that has been retrieved from publicly available proteome databases. For example, polynucleotides encoding long overlapping sequences (e.g., 30 - 100 codons, with 30-50 codon overlap) of the entire human proteome may be designed and integrated in the DNA library.

It is thus preferred that polynucleotides encoding the peptide candidates are introduced into the cell line according to the invention in the form of a library. The peptide candidates encoded in the library preferably have a length of 8-100, 8-90, 8-80, 8-70, 8-60, 8-50, 8-40, 8-30, 8-25, 8-20, 8-15, 8-12 or 8-10 amino acids.

It is preferred herein that the polynucleotides encoding the peptide candidates are transcriptionally linked to a polynucleotide encoding a beta-2 microglobulin as disclosed herein above. It is further preferred that the polynucleotides encoding the beta-2 microglobulin and the polynucleotides encoding the peptide variants are linked by one of the linkers disclosed herein above, in particular linkers encoding a protease-specific cleavage site, a self-cleaving peptide and a signal peptide.

In a first step, a plurality of cells may be obtained by introducing a DNA library into the cell line according to the invention to generate a plurality of cells. It is preferred that at least two cells that are obtained by introducing the DNA library into the cell line according to the invention encode a different peptide candidate. However, more preferably, at least 5, 10, 20, 50, 100, 200, 300, 500, 1'000, 10'000, 100'000 or 1'000'000 cells in the plurality of cells encode a different peptide candidate. Accordingly, in a particular embodiment, the invention relates to the method according to the invention, wherein the plurality of cells encode at least 5, 10, 20, 50, 100, 200, 300, 500, 1'000, 10'000, 100'000 or 1'000'000 different peptide candidate.

The cell line may be any cell line disclosed herein, in particular any cell line comprising a functional IL-2 receptor and a polynucleotide encoding a fluorescent marker under control of the JAK/STAT pathway as disclosed herein. Preferably, the cell line is a cell line in which the endogenous class I HLA genes and, even more preferably, also the endogenous B2M gene, have been inactivated and wherein the cell line has been complemented with a heterologous polynucleotide encoding a class I HLA allele. In embodiments where the endogenous B2M gene has been inactivated, cell line further has to be complemented with a heterologous polynucleotide encoding B2M, which is preferably introduced into the cell line as part of the DNA library. The DNA library may be introduced into the cell line according to the invention as disclosed elsewhere herein.

In a second step, the plurality of cells that has been obtained by introducing the DNA library into the cell line according to the invention may be contacted with a plurality of T cells as disclosed elsewhere herein.

The T cell may be any suitable T cell, such as a CD4+ or a CD8+ T cell. Furthermore, the T cell is required to secrete IL-2 when activated by an antigenic peptide in a TCR-dependent manner. In a preferred embodiment, the T cell is derived from the TnT cell line as disclosed in WO 2021/074249, which is fully incorporated herein by reference.

In a next step, cells presenting peptide candidates that are recognized by the TCR of interest are isolated based on the expression of the fluorescent marker. That is, recognition of a peptide candidate by a T cell results in the activation of the T cell and thus in the secretion of IL-2 by the T cell. The secreted IL-2 can then be recognized by the IL-2 receptor of the antigen-presenting cell according to the invention to induce the expression of the fluorescent marker in a JAK/STAT-dependent manner. Thus, presentation of a peptide candidate that can result in the activation of the T cell will ultimately result in the expression of the fluorescent marker in the cell line according to the invention.

Contacting a plurality of cells presenting different peptide candidates with a plurality of T cells expressing a TCR of interest will result in expression of a fluorescent marker in all cells that present a TCR-recognizable peptide candidate. These cells can then be isolated based on the expression level of the fluorescent marker. Fluorescent cells can readily be separated from non-fluorescent cells, for example by fluorescence-activated cell sorting (FACS). Methods and devices for separating fluorescent cells from non-fluorescent cells are well known in the art. Furthermore, the skilled person is capable of isolating sub-populations of fluorescent cells, i.e. a highly fluorescent sub-population of cells. Thus, in a particular embodiment, the invention relates to the method according to the invention, wherein the cells that express the first fluorescent marker are isolated by fluorescence-activated cell sorting (FACS).

The steps of contacting the cells that present the peptide candidates with T cells may be repeated several times to reduce the risk of obtaining false-positive results. That is, the cells presenting the peptide candidates may be contacted with the T cells for 2, 3, 4, 5 or more times, wherein the fluorescent cells are isolated after each contacting step and contacted with a fresh batch of T cells.

After the final isolation step, the polynucleotides encoding the peptide candidates are sequenced. In certain embodiments, a single cell may be isolated from the population of fluorescent cells and subjected to Sanger sequencing to obtain the nucleotide sequence encoding a peptide candidate that has the potential to activate the TCR of interest. Thus, in a particular embodiment, the invention relates to the method according to the invention, wherein the polynucleotides encoding the peptide candidates are sequenced by Sanger sequencing.

However, it is preferred that that the entire population of fluorescent cells is subjected to deep sequencing as disclosed elsewhere herein. Thus, in a particular embodiment, the invention relates to the method according to the invention, wherein the polynucleotides encoding the peptide candidates are sequenced by deep sequencing.

Deep sequencing results in a large number of reads which may be assigned to specific peptide candidates, preferably by using bioinformatic methods known in the art. Peptide candidates that can activate a TCR of interest can then be identified by analysing the abundance of reads encoding these peptide candidates.

Preferably, peptide candidates that are recognized by a TCR of interest are identified by read enrichment analysis. Thus, in a particular embodiment, the invention relates to the method according to the invention, wherein targets of the TCR of interest are identified by read enrichment analysis of the deep sequencing results.

For that, it is preferred that at least two populations of cells are subjected to deep sequencing. The first population is the fluorescent population of cells that has been obtained after the final isolation step. That is, the first population of cells is a population in which TCR-recognizable peptide candidates have been enriched. The second population of cells is a non-fluorescent population, preferably from the same batch from which the fluorescent population has been isolated.

By comparing the deep sequencing results of the two populations it is possible to determine the enrichment factor of each peptide candidate in the library. Peptide candidates that are enriched in the fluorescent population can then be identified as peptide candidates that can activate the TCR of interest, and consequently as targets of the TCR of interest.

Accordingly, any peptide candidate in the fluorescent population, irrespective whether it has been identified by Sanger sequencing of deep sequencing, can be considered a target of the TCR of the interest.

In a particular embodiment, the invention relates to the method according to the invention, the method comprising a further step of predicting targets of the TCR of interest by applying a machine learning model to a human peptidome database, wherein the machine learning model has been trained with the deep sequencing data.

In order to expand the limits of experimental T cell antigen screening, read frequency enrichment data from fluorescent and non-fluorescent population may be encoded and used to train supervised machine learning models that can perform classification of the screened TCR for activation or non-activation based on a given antigen sequence. The skilled person is aware of methods for encoding read frequency enrichment data. In certain embodiments, read frequency enrichment data may be encoded by one-hot encoding as known in the art. The machine learning model may be any suitable machine learning model known in the art, including, without limitation, a support vector machine (SVM) algorithm, a random forest algorithm, or an artificial neural network. These models are then applied to the entire human peptidome in order to assign every candidate antigen sequence with a probability of TCR activation, thus enabling comprehensive discovery of T cell antigens that activate the candidate TCR.

In a particular embodiment, the invention relates to a method for assessing the alloreactivity of a T cell receptor (TCR), the method comprising the steps of: (a) providing a plurality of cells according to the invention, wherein the plurality of cells encode at least one heterologous HLA allele; (b) contacting the plurality of cells of step (a) with a plurality of T cells; (c) isolating cells that express the first fluorescent marker; (d) sequencing the heterologous polynucleotides encoding the HLA alleles in the cells that have been isolated in step (c); and (e) identifying an HLA molecule as a target of an alloreactive TCR based on the sequencing results that have been obtained in step (d).

That is, the cell line according to the invention may not only be used for identifying targets or off-targets of TCRs but may also be used for identifying alloreactive TCRs. Alloreactive TCRs are TCRs that have the potential to recognise a particular HLA allele irrespective of which peptide is presented. In particular when engineering TCR for a specific antigen, it is important to ensure that the engineered TCR is not alloreactive.

The alloreactivity of a TCR may be assessed in the cell line according to the invention. In particular, the alloreactivity of a TCR may be assessed in a cell line comprising an IL-2 receptor and a polynucleotide encoding a fluorescent marker under control of the JAK/STAT signalling pathway. Furthermore, the cell line is a cell line in which all endogenous class I and/or class II HLA genes have been inactivated.

In certain embodiments, the cell line according to the invention has been complemented with a polynucleotide encoding a heterologous HLA allele. In such embodiments, it may be assessed whether a specific TCR or a library of TCRs is alloreactive for said HLA allele. However, it is preferred herein that the plurality of cells is obtained by introducing a library of different HLA alleles into the cell line according to the invention. That is, in a particular embodiment, the invention relates to the method according to the invention, wherein at least two cells in the plurality of cells of step (a) encode a different HLA allele. In a preferred embodiment, the invention relates to the method according to the invention, wherein at least 5, 10, 25, 50, 75, 100, 150, 200, 300, 400, 500, 1'000, 2'500, 5'000, 10'000 or 25'000 cells in the plurality of cells of step (a) encode a different HLA allele.

In certain embodiments, the plurality of cells may encode heterologous HLA class I alleles. In certain embodiments, the plurality of cells may encode heterologous HLA class II alleles. In certain embodiments, the plurality of cells may encode heterologous HLA class I and class II alleles. The HLA alleles that are introduced into the cell line according to the invention may be derived from publicly available HLA databases, such as https://www.ebi.ac.uk/ipd/imgt/hla/. Further, the skilled person is aware of methods to generate libraries encoding a plurality of HLA alleles and introducing such libraries into a cell line according to the invention to obtain the plurality of cells. In certain embodiments, the plurality of cells may encode the HLA class I and or class II alleles that are most common in humans.

Once designed, gene cassettes encoding individual HLA alleles may be generated by gene synthesis and cloned as a pool into a plasmid containing homology arms mapping to a locus in the cell line according to the invention. HDR templates may then be generated by PCR and the resulting amplicons may be electroporated into the cell line according to the invention.

In a next step, the plurality of cells expressing the heterologous HLA alleles may be contacted with a plurality of T cells.

In certain embodiments, the plurality of T cells may be a population of T cells expressing an identical TCR. Thus, in certain embodiments, the invention relates to the method according to the invention, wherein the T cells express an identical TCR. The identical TCR may be a naturally occurring TCR or an engineered TCR. For example, it may be assessed with a library encoding a plurality of HLA alleles, whether an engineered TCR is alloreactive. When contacting a population of T cells expressing an identical TCR with a population of cells expressing one or more heterologous HLA alleles, it is possible to identify at an early stage in T cell discovery whether a TCR has the potential to be alloreactive and against which HLA alleles the TCR may be alloreactive. By using a library encoding the most common human HLA alleles, it is possible to use the method according to the invention for eliminating TCRs from the discovery pipeline that are potentially alloreactive.

In certain embodiments, the plurality of T cells may be a population of T cells expressing different TCRs. Thus, in a particular embodiment, the invention relates to the method according to the invention, wherein at least two T cells in the plurality of T cells express different TCRs. For example, the population of T cells may have been obtained by introducing a library encoding different TCRs into a population of cells. This population of T cells may then be contacted with a population of cells according to the invention. The population of cells according to the invention may encode a single heterologous HLA allele. In such embodiments, it may be assessed whether one or more of the TCRs encoded in the population of T cells is alloreactive for that specific HLA allele. In certain embodiments, the population of cells according to the invention may encode a plurality of HLA alleles. In such embodiments, it may be assessed whether a plurality of TCRs comprises any alloreactive TCRs.

In a particular embodiment, the invention relates to the method according to the invention, wherein at least 5, 10, 20, 30, 40, 50, 60, 70, 80, 90 or 100 T cells in the plurality of T cells express different TCRs.

The plurality of T cells may be a plurality of naturally occurring T cells, for example a population of T cells that have been isolated from a blood sample. In certain embodiments, the population of T cells may be derived from a T cell line, such as, without limitation, a Jurkat cell line. To obtain a population of T cells expressing different TCRs, a TCR-encoding DNA library may be introduced into a T cell line, such as a Jurkat cell line.

In a preferred embodiment, the T cell may be the TnT cell line as disclosed in WO 2021/074249 or may be derived from the TnT cell line. The TnT cell line has the advantage that it comprises a second fluorescence marker that is expressed when the T cell is activated. Thus, displaying a suitable antigen on the cell line according to the invention in the presence of a TnT cell expressing a cognate TCR will result in the expression of the first fluorescent marker in the cell line according to the invention and in the expression of the second fluorescent marker in the TnT cell line. Thus, by expressing an HLA-encoding DNA library in the cell line according to the invention and a TCR-encoding DNA library in the TnT cell line, it is not only possible to identify HLA alleles that can be recognized by alloreactive TCRs, but it also allows identifying the population of alloreactive TCRs.

Thus, in a particular embodiment, the invention relates to the method according to the invention, wherein the T cell is an engineered T cell, in particular wherein the engineered T cell comprises a polynucleotide encoding a second fluorescent marker under control of an NFAT transcription factor.

That is, the T cell may be a cell that comprises a TCR and secretes IL-2 when activated. Preferably, the T cell comprises a reporter system that additionally results in the expression of a fluorescent marker when the T cell is activated in a TCR-dependent manner. For that, it is preferred that the polynucleotide encoding the fluorescent marker is under control of a promoter comprising at least one NFAT transcription factor response element. Most preferably, the engineered T cell is the TnT cell line as disclosed in WO 2021/074249, which is fully incorporated by reference herein.

In certain embodiments, the cell line according to the invention expresses a first fluorescent marker and the T cell expresses a second fluorescent marker. It is preferred herein that the first and second fluorescent marker are compatible, i.e. that there is no significant crosstalk between the two markers. In certain embodiments, one fluorescent marker is a red fluorescent protein, such as, without limitations, mRuby2, and the other fluorescent marker is a green fluorescent protein, such as, without limitation, GFP.

As disclosed above for the other methods of the invention, the step of contacting the cell line according to the invention with the T cells may be repeated 2, 3, 4, 5, or more times in order to improve the quality of the results.

As mentioned above, the method of the invention may comprise an additional step of isolating activated T cells based on the expression of the second fluorescent marker. The isolated T cells may then be subjected to sequencing to identify the amino acid sequence of their T cell receptors. Thus, in a particular embodiment, the invention relates to the method according to the invention, the method comprising further steps of (f) isolating T cells that express the second fluorescent marker; (g) sequencing the polynucleotides encoding the TCRs in T cells that have been isolated in step (f); and (h) identifying a TCR as an alloreactive TCR based on the sequencing results that have been obtained in step (g).

Both the cell according to the invention expressing the first fluorescent marker and the T cell expressing the second fluorescent marker are preferably isolated by FACS as described herein. Thus, in a particular embodiment, the invention relates to the method according to the invention, wherein the cells that express the first fluorescent marker, and optionally the second fluorescent marker, are isolated by fluorescence-activated cell sorting (FACS).

Identifying HLA alleles that can be recognized by an alloreactive TCR or identifying the alloreactive TCR itself preferably comprises a step of sequencing the polynucleotides encoding the HLA alleles and/or the TCRs in the fluorescent cells. In certain embodiments, the polynucleotides encoding the HLA alleles and/or the TCRs may be isolated from fluorescent cells and subjected to Sanger sequencing as known in the art to retrieve the nucleotide sequences of the HLA alleles and/or the TCRs. Thus, in a particular embodiment, the invention relates to the method according to the invention, wherein the polynucleotides encoding the HLA alleles, and optionally the TCRs, are sequenced by Sanger sequencing.

However, it is preferred that the polynucleotides encoding the HLA alleles and/or the TCRs are sequenced by deep sequencing as described elsewhere herein. Thus, in a particular embodiment, the invention relates to the method according to the invention, wherein the polynucleotides encoding the HLA alleles, and optionally the TCRs, are sequenced by deep sequencing.

It is important to note that deep sequencing reads are typically too short to cover an entire polynucleotide encoding an HLA allele or a TCR. However, bioinformatic methods are known in the art that allow assigning the obtained reads to a respective HLA allele. All reads that cannot be assigned to a specific HLA allele may not be taken into account for the analysis. For the identification of alloreactive TCRs with the method according to the invention, sequencing may be restricted to the complementary determining regions of the TCR, which are short enough to be covered by deep sequencing reads.

In a particular embodiment, the invention relates to the method according to the invention, wherein HLA molecules are identified as a target of an alloreactive TCR, and/or wherein TCRs are identified as alloreactive TCRs by read enrichment analysis of the deep sequencing results.

As mentioned above, the reads obtained by deep sequencing may be assigned to a specific HLA allele or a specific TCR. To assess whether an HLA allele is recognized by an alloreactive TCR or whether a TCR is alloreactive, the deep sequencing reads may be analysed by read enrichment analysis. That is, after contacting the cell according to the invention with the T cells, both non-fluorescent populations of cells and fluorescent populations of cells may be isolated and subjected to deep sequencing. Subsequently, it may be analysed for each HLA allele or each TCR by which factor the corresponding reads are enriched in the fluorescent cell populations.

### BRIEF DESCRIPTION OF THE FIGURES

**FIG.1****:** Overview of the TCR-Safe workflow for cross-reactivity screening and target discovery. TCR-Safe combines CRISPR-targeted mammalian display (ACDC cells), functional screening (ACDC and TnT-TCR co-culture), FACS, deep sequencing and computational analysis for the functional screening of TCR antigen libraries at high-throughput.
**FIG.2****:** Overview of the TCR-Safe workflow for alloreactivity screening. TCR-Safe combines CRISPR-targeted mammalian display (ACDC cells), functional screening (ACDC and TnT-TCR co-culture), FACS, deep sequencing and computational analysis for the functional screening of HLA allele libraries at high-throughput.

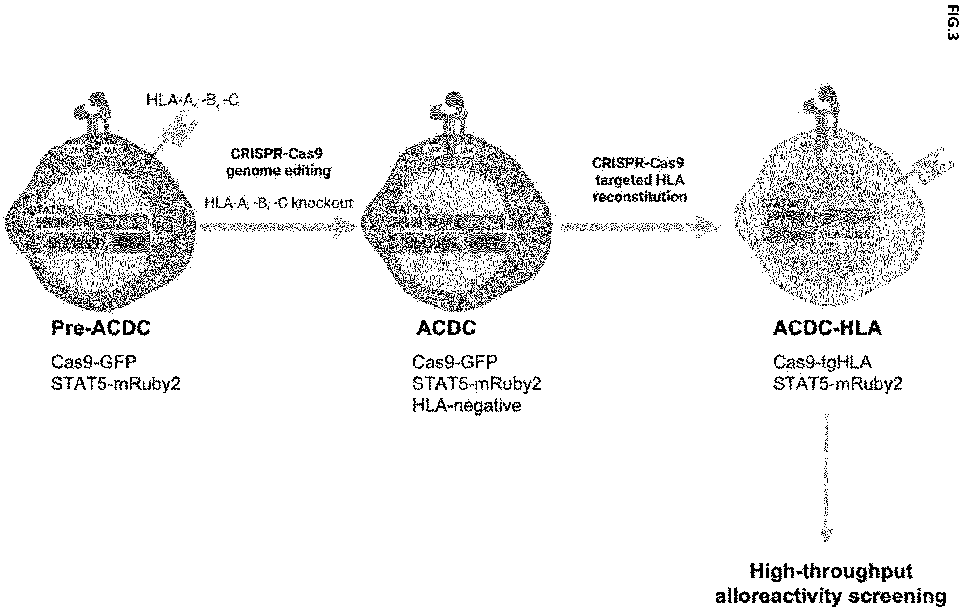

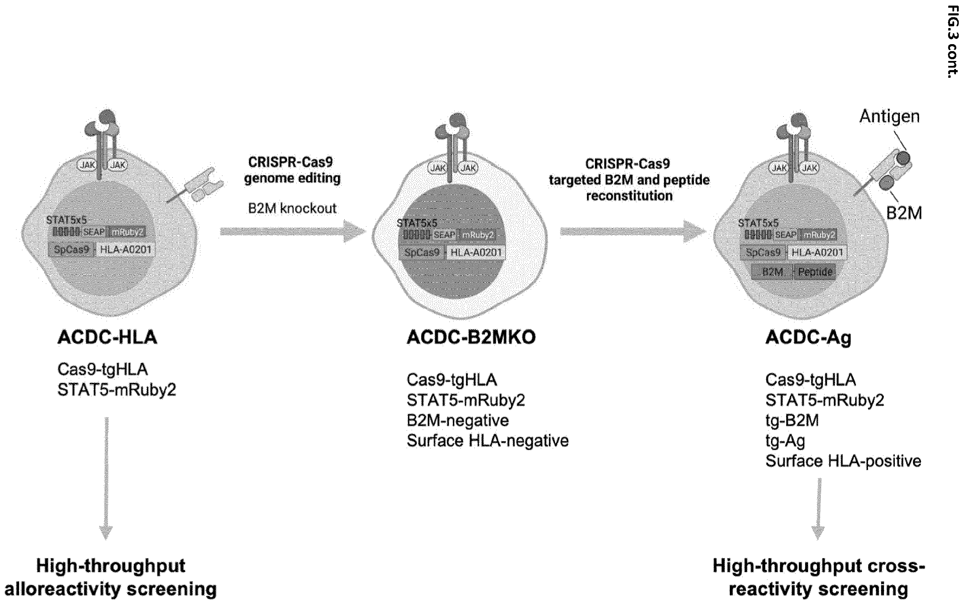

**FIG.3****:** Schematic representation of the multiple CRISPR-Cas9 genome editing steps enabling functional display of HLA and TCR antigen libraries by ACDC cells.

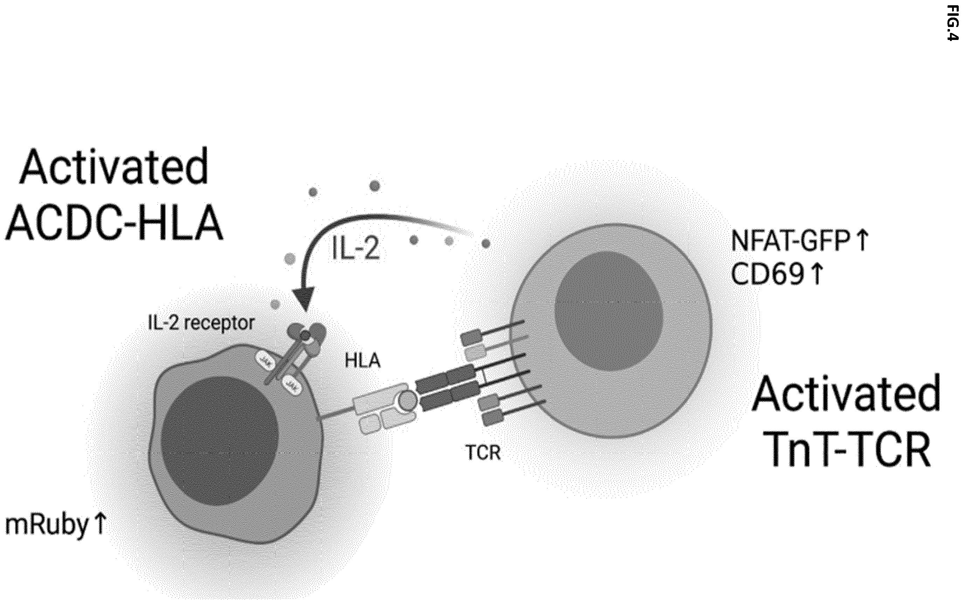

**FIG.4****:** Schematic representation of IL-2 detection by ACDC cells following TCR antigen recognition. ACDC cells displaying a TCR antigen (orTCR antigen libraries) are co-cultured with TnT cells displaying a TCR of interest. Following antigen recognition, activated TnT-TCR cells locally secrete IL-2 that is detected by the activating ACDC-HLA cell expressing the trimeric high-affinity IL-2 receptor and harbouring a STAT5-mRuby2 fluorescent reporter of IL-2 signalling.

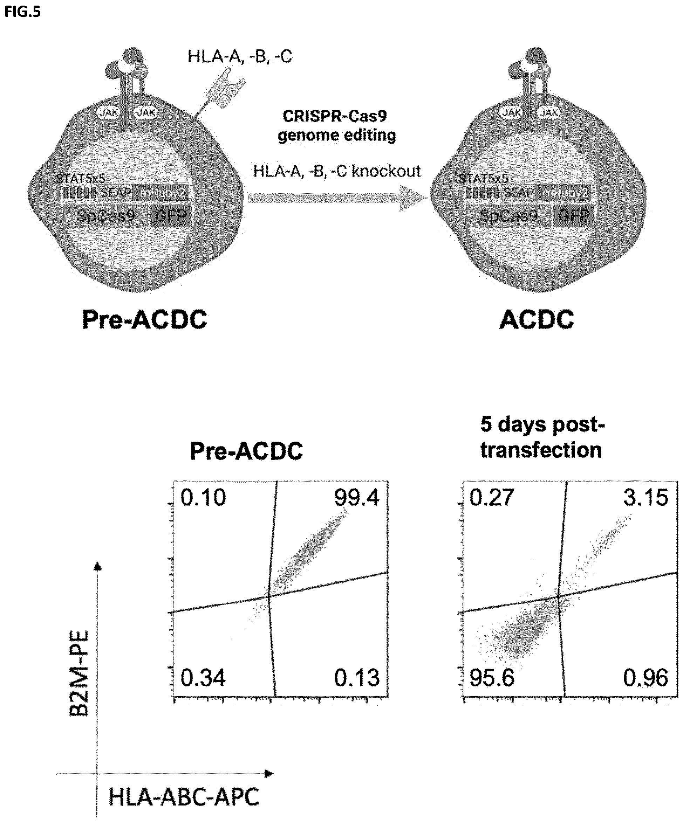

**FIG.5****:** CRISPR-targeted knockout of endogenous HLA class I genes in pre-ACDC cells. Pre-ACDC cells (Cas9-GFP+, IL-2R+, JAK3+, STAT5-mRuby2 reporter) were subjected to CRISPR-Cas9 genome editing in order to disrupt the expression of endogenous HLA class I genes (upper panel). A pan-HLA class I gRNA targeting a highly conserved region (exon 4) was designed and delivered into pre-ACDC cells by means of electroporation. Flow cytometry plots at 5 days post transfection reveal highly efficient disruption of HLA-A, HLA-B and HLA-C surface expression (> 95% knockout rate, lower panel).

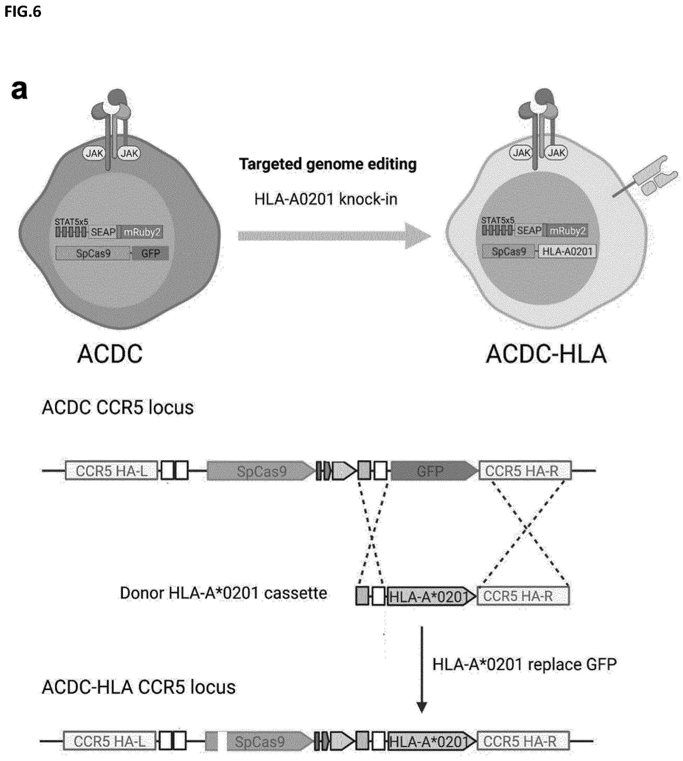

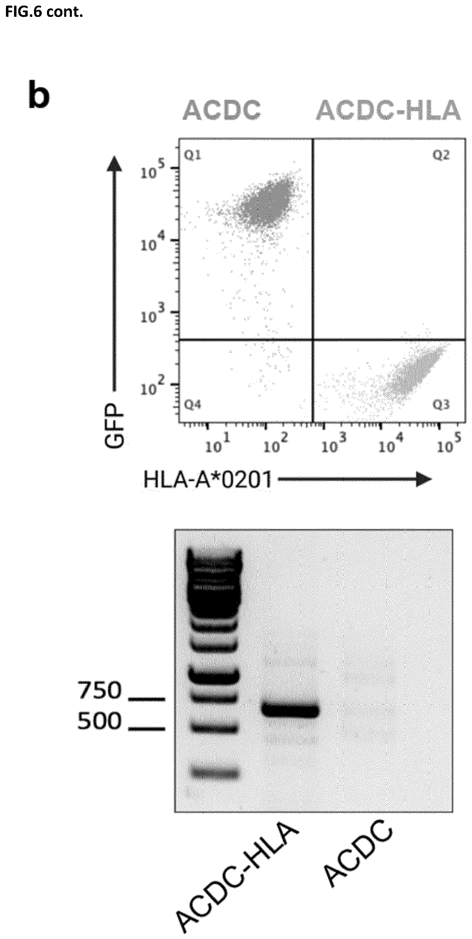

**FIG.6****:** CRISPR-targeted integration of transgenic HLA-A^{∗}0201 in ACDC cells. a. The GFP transgene (CCR5 locus) of ACDC cells (Cas9-GFP+, IL-2R+, JAK3+, STAT5-mRuby2 reporter, HLA-I-negative) was subjected CRISPR-targeted replacement using a homology directed repair (HDR) template encoding the HLA-A^{∗}0201 allele. b. Assessment of GFP and HLA-A^{∗}0201 expression by means of flow cytometry (top) and genomic PCR of the CCR5 locus (bottom) of ACDC and ACDC-HLA cells reveal successful CRISPR-targeted replacement of GFP for HLA-A^{∗}0201.

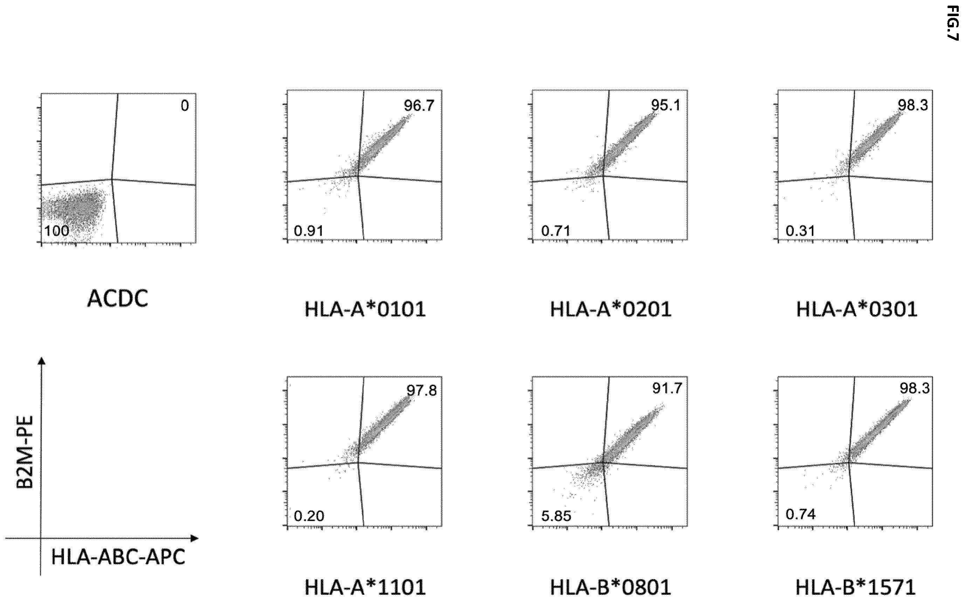

**FIG.7****:** CRISPR-targeted integration of transgenic HLA class I alleles in ACDC cells (panel 1). ACDC cells were subjected to CRISPR-targeted replacement of the GFP transgene for A^{∗}0101, A^{∗}0201, A^{∗}0301, A^{∗}1101, B^{∗}0801 or B^{∗}1571 HLA class I alleles. Flow cytometry plots show successful HLA integration as assessed by B2M and HLA-A/B/C surface expression.

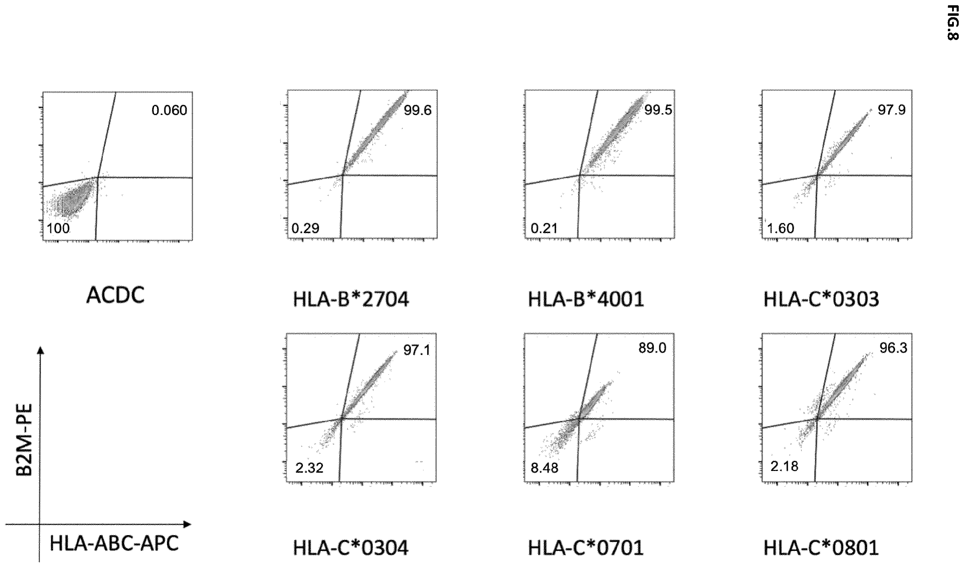

**FIG.8****:** CRISPR-targeted integration of transgenic HLA class I alleles in ACDC cells (panel 2). ACDC cells were subjected to CRISPR-targeted replacement of the GFP transgene for B^{∗}2704, B^{∗}4001, C^{∗}0303, C^{∗}0304, C^{∗}0701 or C^{∗}0801 HLA class I alleles. Flow cytometry plots show successful HLA integration as assessed by B2M and HLA-A/B/C surface expression.

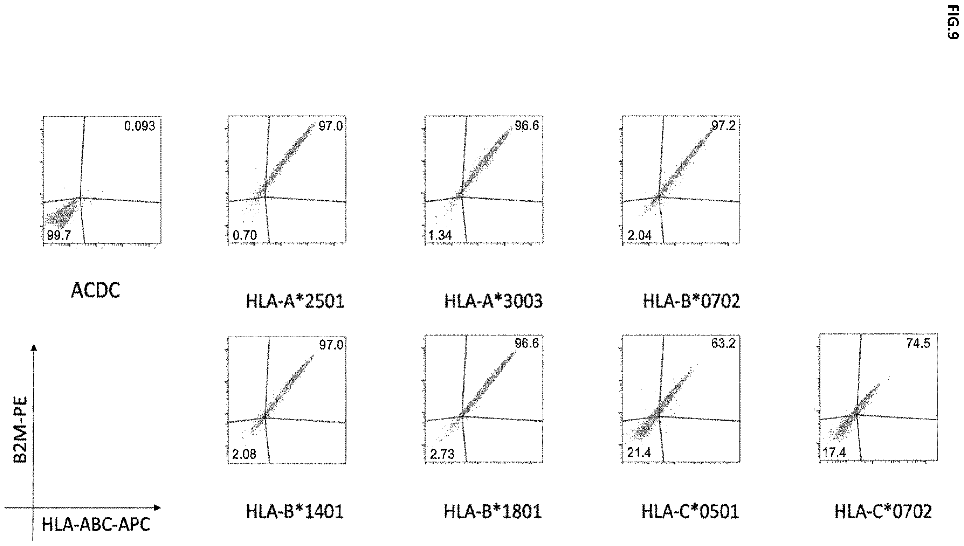

**FIG.9****:** CRISPR-targeted integration of transgenic HLA class I alleles in ACDC cells (panel 3). ACDC cells were subjected to CRISPR-targeted replacement of the GFP transgene for A^{∗}2501, A^{∗}3003, B^{∗}0702, B^{∗}1401, B^{∗}1801, C^{∗}0501 or C^{∗}0702 HLA class I alleles. Flow cytometry plots show successful HLA integration as assessed by B2M and HLA-A/B/C surface expression.

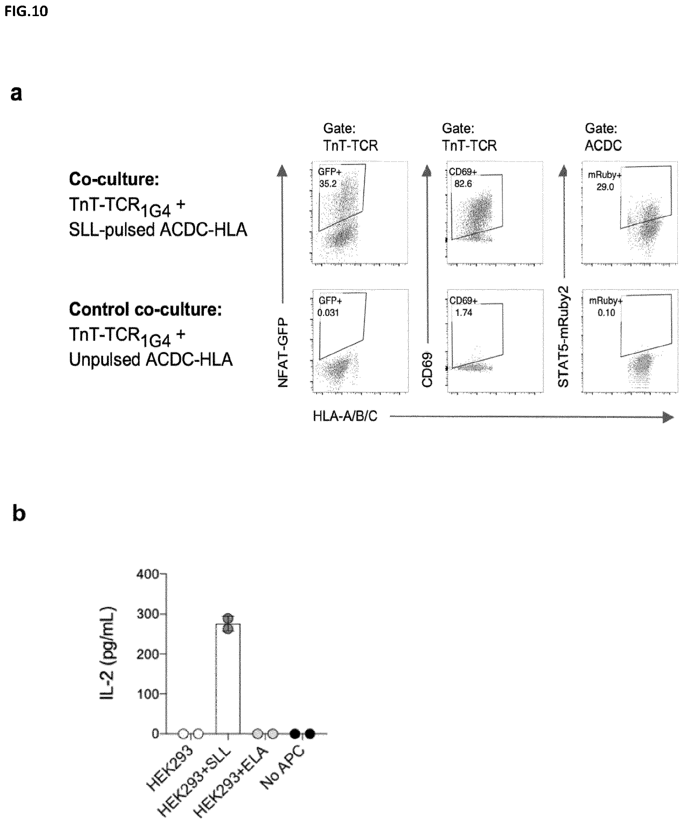

**FIG.10****:** Functional validation of ACDC platform using peptide-pulsed ACDC-HLA cells. a. TnT cells displaying TCR1G4 specific for the NY-ESO-1₁₅₇₋₁₆₅ SLLMWITQC antigen (SEQ ID NO: 10) were co-cultured overnight with SLLMWITQC-pulsed (SEQ ID NO: 10) or unpulsed ACDC-HLA (A^{∗}0201) cells. Flow cytometry analysis confirms antigen-specific activation of both TnT-TCR1G4 cells (NFAT-GFP and CD69 expression) and ACDC-HLA cells (STAT5-mRuby2 expression) in the presence of SLLMWITQC peptide (SEQ ID NO: 10) only. b. TnT-TCR1G4 cells were co-cultured overnight with SLLMWITQC-pulsed (SEQ ID NO: 10), ELAGIGILTV-pulsed (SEQ ID NO:11) or unpulsed HEK293 cells (ACDC progenitor), as well as in the absence of HEK293. IL-2 ELISA of culture supernatants confirmed exclusive activation of TnT-TCR1G4 cells in the presence of cognate peptide (SLLMWITQC; SEQ ID NO: 10).

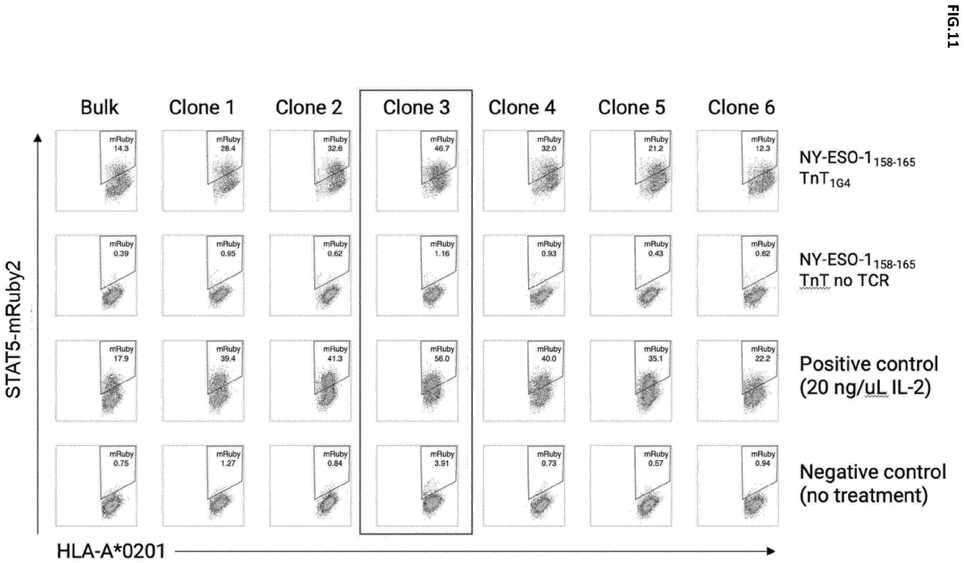

**FIG.11****:** Selection of an ACDC-HLA (A^{∗}0201) clone with high IL-2 sensitivity. ACDC-HLA (A^{∗}0201) cells were subjected to single-cell FACS in order to derive monoclonal cell lines. Following expansion in culture, clones were pulsed with SLLMWITQC peptide (SEQ ID NO: 10) and: (i) co-cultured overnight with TnT-TCR1G4 cells; (ii) co-cultured overnight with TnT cells (no TCR); (iii) stimulated overnight with soluble human IL-2; or (iv) received no further treatment. Assessment of STAT5-mRuby2 expression by flow cytometry identified 'Clone 3' showing robust activation in response to both TnT-TCR1G4-secreted and recombinant soluble IL-2.

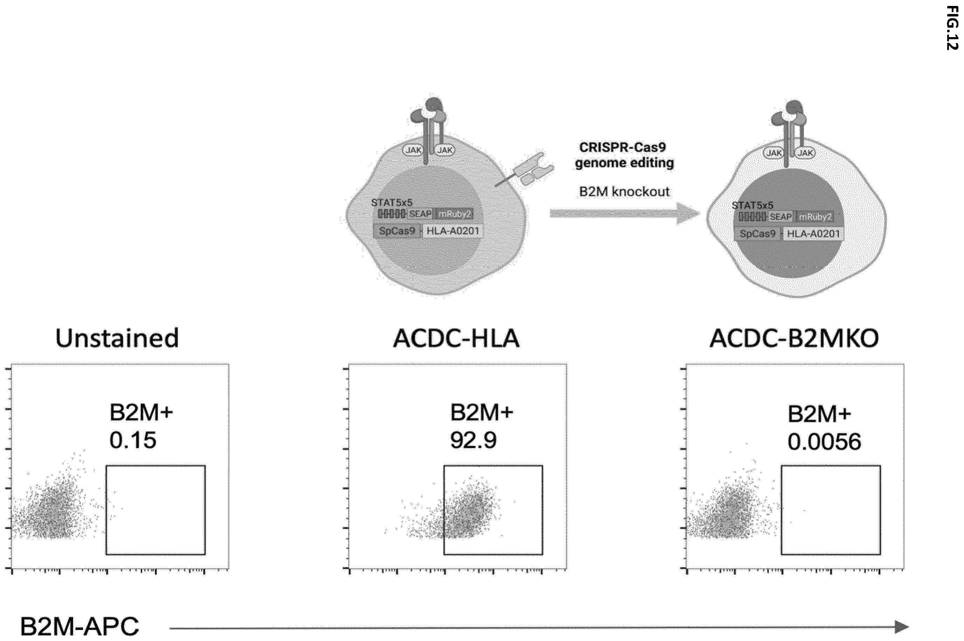

**FIG.12****:** CRISPR-targeted knockout of the endogenous beta-2-microglobulin (B2M) gene in ACDC-HLA cells. ACDC-HLA cells (Cas9-tgHLA-A^{∗}0201+, IL-2R+, JAK3+, STAT5-mRuby2 reporter) were subjected to CRISPR-Cas9 genome editing in order to disrupt the expression of the endogenous B2M gene. A gRNA targeting B2M exon 2 was designed and delivered into ACDC-HLA cells by means of electroporation. Following expansion in culture, B2M-negative cells were subjected to two rounds of FACS enrichment in order to obtain pure populations of ACDC-B2MKO cells.

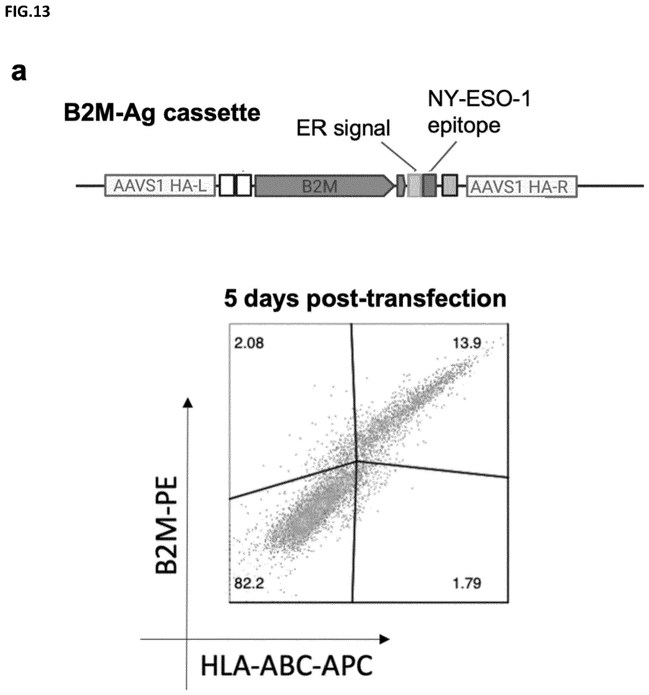

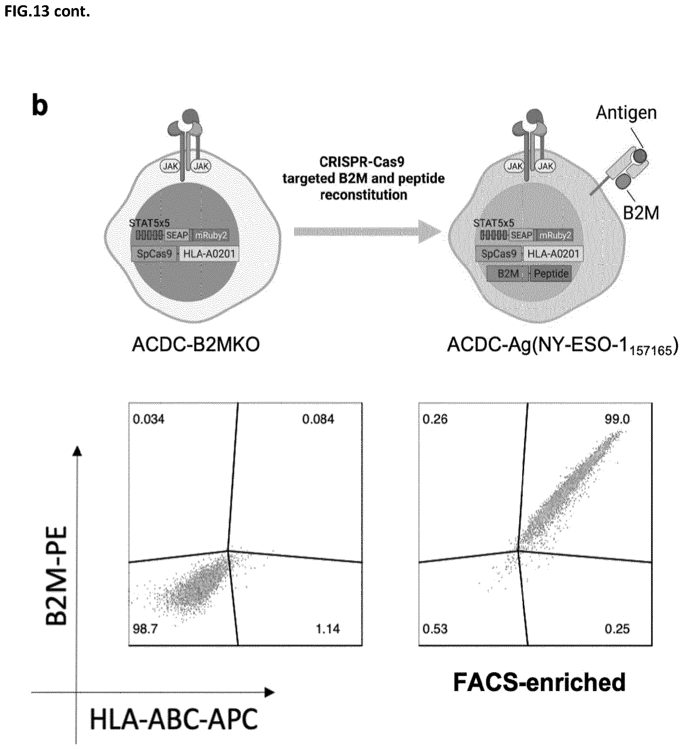

**FIG.13****:** CRISPR-targeted reconstitution of B2M expression for TCR antigen display. a. Schematic representation of B2M-Ag HDR template design for CRISPR-Cas9 integration targeted to the AAVS1 safe harbour locus (top). ACDC-HLA (A^{∗}0201) cells were electroporated with B2M-Ag HDR template by means of electroporation and expanded in culture. Flow cytometry analysis of B2M and HLA-A/B/C surface expression at 5 days post-transfection confirms successful integration of the B2M-Ag cassette at 13.9% HDR efficiency (bottom). b. Following expansion in culture, B2M-positive, HLA-A/B/C-positive cells were subjected to two rounds of FACS enrichment in order to obtain pure populations of ACDC-Ag cells.

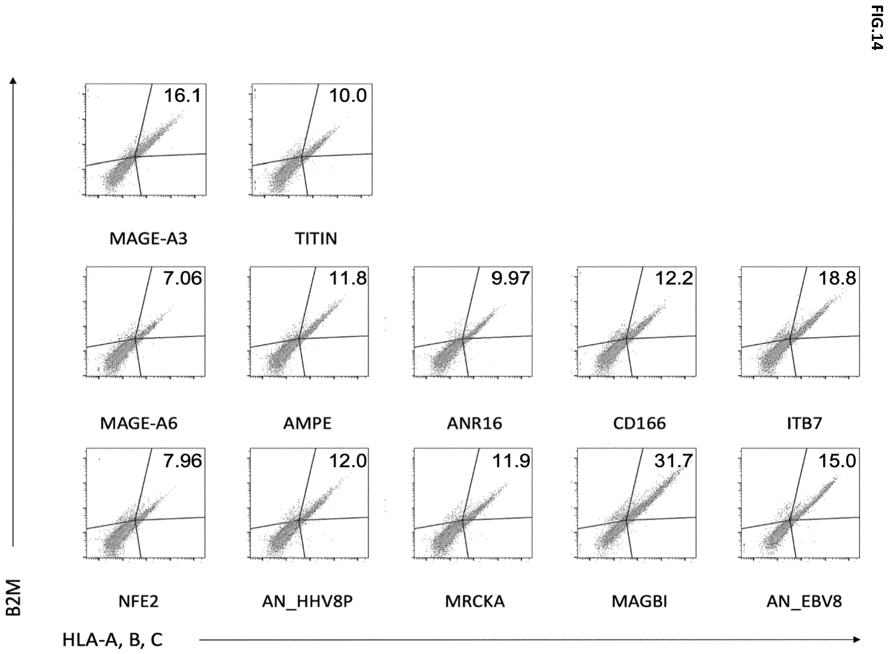

**FIG.14****:** Display of multiple candidate T cell antigens by CRISPR-targeted reconstitution of B2M expression. B2M-Ag cassettes encoding multiple HLA-A^{∗}0101-restricted TCR antigens were designed and generated by gene synthesis. HDR templates were generated by PCR amplification and delivered in to ACDC-HLA (A^{∗}0101) cells by means of electroporation. Flow cytometry analysis of B2M and HLA-A/B/C surface expression at 5 days post-transfection confirms successful integration of the B2M-Ag cassettes at 7% to 31% HDR efficiencies.

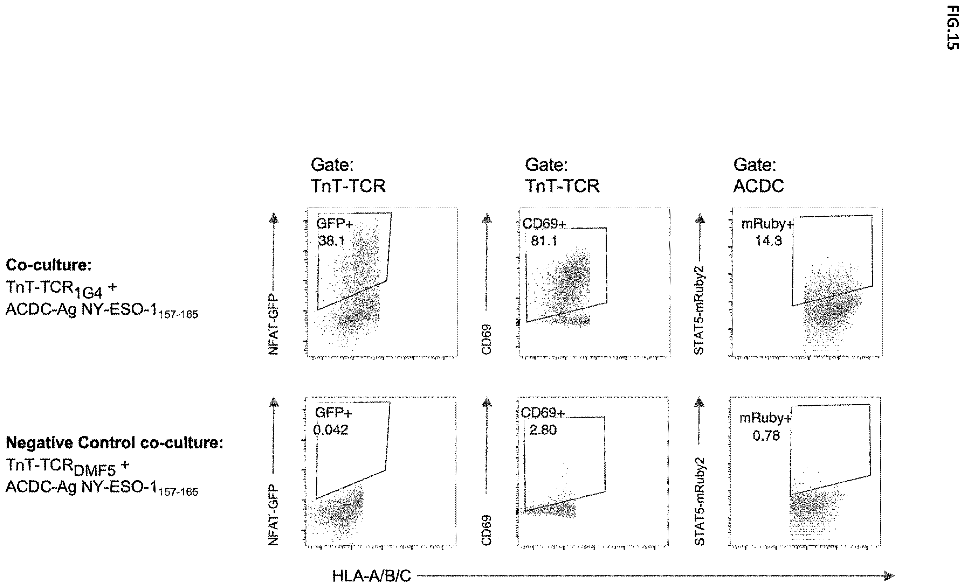

**FIG.15****:** Functional validation of ACDC-Ag cells expressing genomically-encoded TCR antigens. TnT cells displaying TCR1G4 (specific NY-ESO-1₁₅₇₋₁₆₅ SLLMWITQC (SEQ ID NO: 10)) or TCRDMF5 (specific for MART-1₂₆₋₃₅(27L) ELAGIGILTV (SEQ ID NO: 11)) were co-cultured overnight with ACDC-Ag cells expressing HLA-A^{∗}0201 and NY-ESO-1₁₅₇₋₁₆₅ SLLMWITQC antigen (SEQ ID NO: 10). Flow cytometry analysis shows robust activation of TnT-TCR (NFAT-GFP and CD69) and ACDC-Ag cells (STAT5-mRuby2) only when TnT-TCR1G4 are used as effectors.

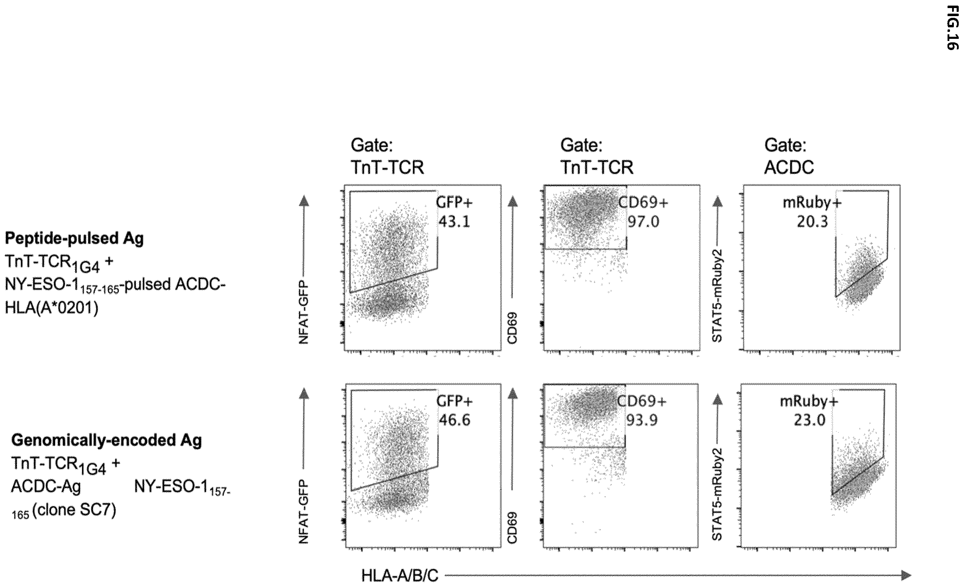

**FIG.16****:** Genomically-encoded T cell antigens induce robust TnT-TCR and ACDC-Ag activation. TnT cells displaying TCR1G4 specific for the NY-ESO-1₁₅₇₋₁₆₅ SLLMWITQC (SEQ ID NO: 10) were co-cultured overnight with SLLMWITQC-pulsed (SEQ ID NO: 10) ACDC-HLA (A^{∗}0201) cells or with a highly responsive clone (SC7) of ACDC-Ag cells expressing HLA-A^{∗}0201 and NY-ESO-1₁₅₇₋₁₆₅ SLLMWITQC antigen (SEQ ID NO: 10). Flow cytometry analysis shows similar activation levels of TnT-TCR (NFAT-GFP and CD69) and ACDC-Ag cells (STAT5-mRuby2) in response to peptide-pulsed and genomically-encoded NY-ESO-1₁₅₇₋₁₆₅ SLLMWITQC antigen (SEQ ID NO: 10).
**FIG.17****:** Design and CRISPR-targeted integration of a MAGE-A3₁₆₈₋₁₇₆ positional scanning library (SEQ ID NO:12-31) into ACDC-HLA cells. a. Schematic representation of a MAGE-A3₁₆₈₋₁₇₆ EVDPIGHLY positional scanning library encoded within the B2M-Ag gene cassette for CRISPR-Cas9 integration targeted to the AAVS1 safe harbour locus. b. HDR templates were generated by PCR amplification and delivered into ACDC-HLA (A^{∗}0101) cells by means of electroporation. Flow cytometry analysis of B2M and HLA-A/B/C surface expression at 5 days post-transfection confirmed successful integration of the B2M-Ag library at 10.1% HDR efficiency. c. Following expansion in culture, transfected cells were subjected to two rounds of FACS enrichment and genomic PCR amplification of the transgenic B2M-Ag locus. PCR amplicons were cloned into the pYTK001 plasmid and transformed into chemically competent E. coli cells. Sanger sequencing of plasmids derived from eight transformants identified 6 transformants harbouring single codon mutants (SEQ ID NO:32-37) and one transformant harbouring the wild-type MAGE-A3₁₆₈₋₁₇₆ EVDPIGHLY T cell antigen (SEQ ID NO:12).
**FIG.18****:** Overview of candidate T cell antigen library design and their application for TCR-Safe cross-reactivity screening and target discovery. Schematic representation of the types of TCR antigen libraries displayed on ACDC-Ag cells for high-throughput functional screening. Positional scanning libraries (SEQ ID NO:10 and 38-56) consisting of every possible single amino acid mutant of the intended target are applied for TCR specificity profiling and generation of sequence motifs used for prediction of potential TCR off-targets from proteome database searches. Combinatorial libraries of moderate diversity (hundreds to tens of thousands of variants) are designed to encode individual potential off-targets originating from positional scanning predictions or existing human sequences with ≥ 50% similarity to the intended target. Combinatorial libraries of high diversity (hundreds of thousands to billions of variants) are computationally designed using degenerate codons to mimic amino acid frequencies derived from publicly available HLA binding motifs. A second type of high diversity combinatorial library is computationally designed to incorporate DNA oligonucleotides encoding overlapping polypeptides covering the entire human proteome.

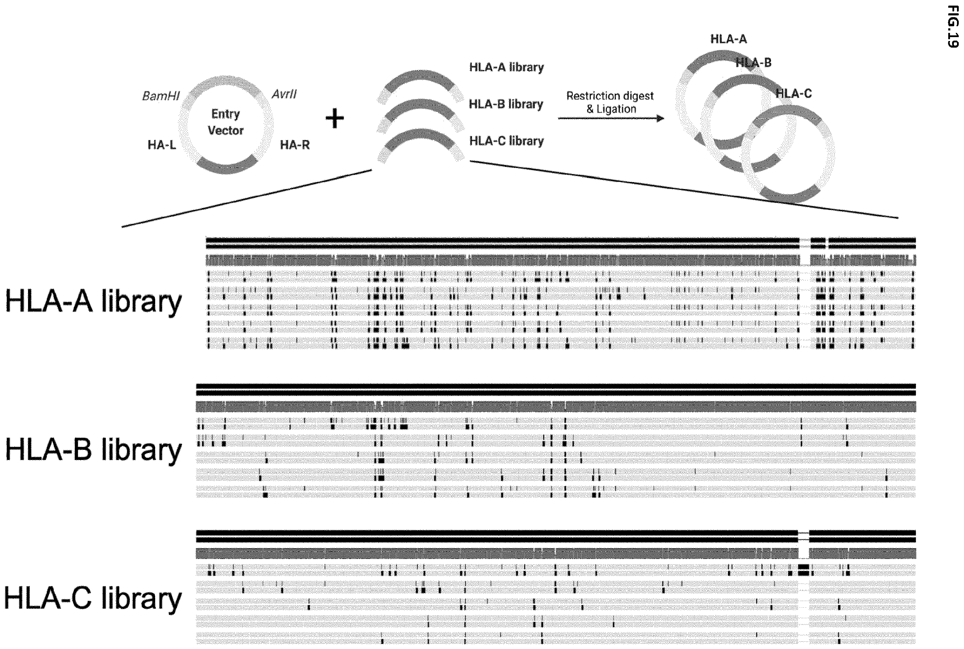

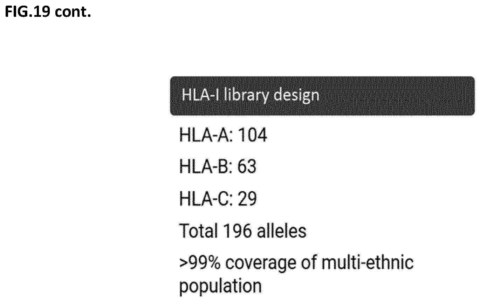

**FIG.19****:** Overview of HLA class I allele library design and assembly. A library encoding 196 HLA class I alleles is designed to incorporate commonly occurring alleles across major human populations (> 99% cumulative frequency per gene). The HLA-I library is generated by gene synthesis and introduced as a pool into a donor plasmid containing a 5' homology arm mapping to the region upstream of the GFP transgene and a 3' homology arm mapping to the CCR5 by means of BamHI and AvrII restriction cloning.

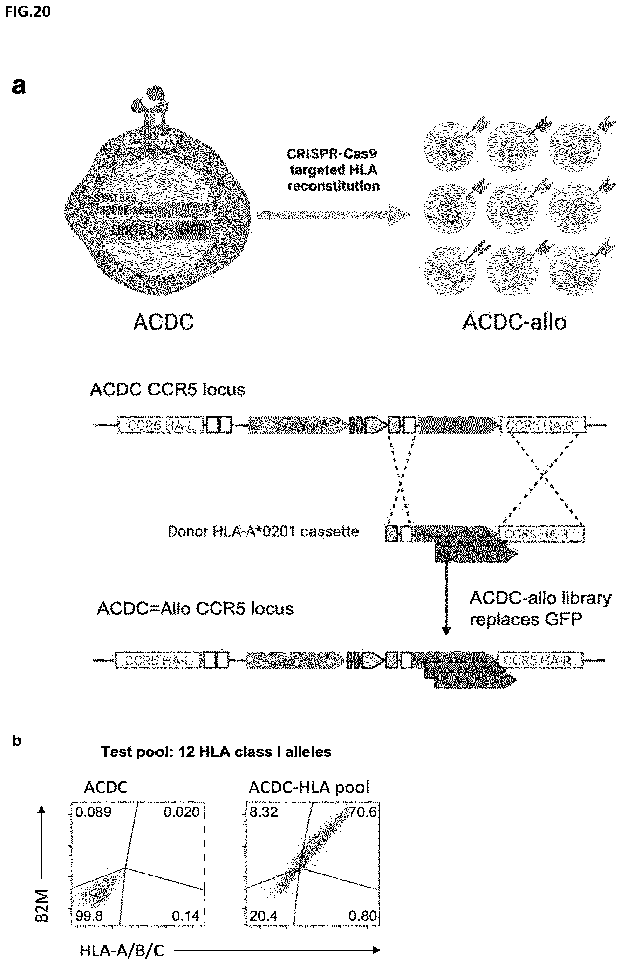

**FIG.20****:** CRISPR-targeted display of transgenic HLA class I allele libraries in ACDC cells. a. The GFP transgene (CCR5 locus) of ACDC cells (Cas9-GFP+, IL-2R+, JAK3+, STAT5-mRuby2 reporter, HLA-I-negative) is subjected CRISPR-targeted replacement using a homology directed repair (HDR) template encoding the pool of HLA class I alleles. b. Assessment of B2M and HLA-A/B/C expression by means of flow cytometry shows successful CRISPR-targeted integration of an HLA-I library containing 12 HLA class I alleles.

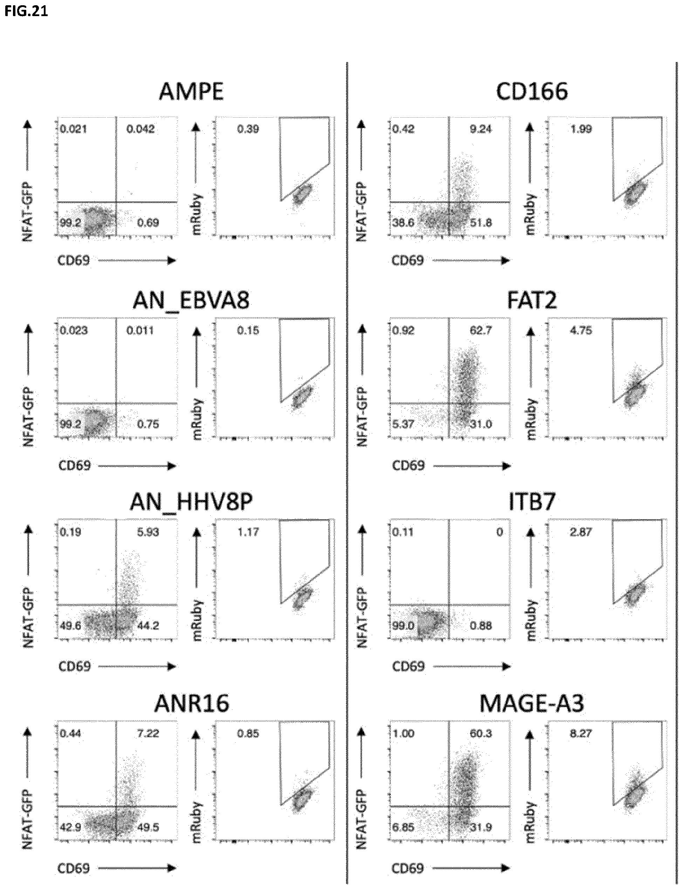

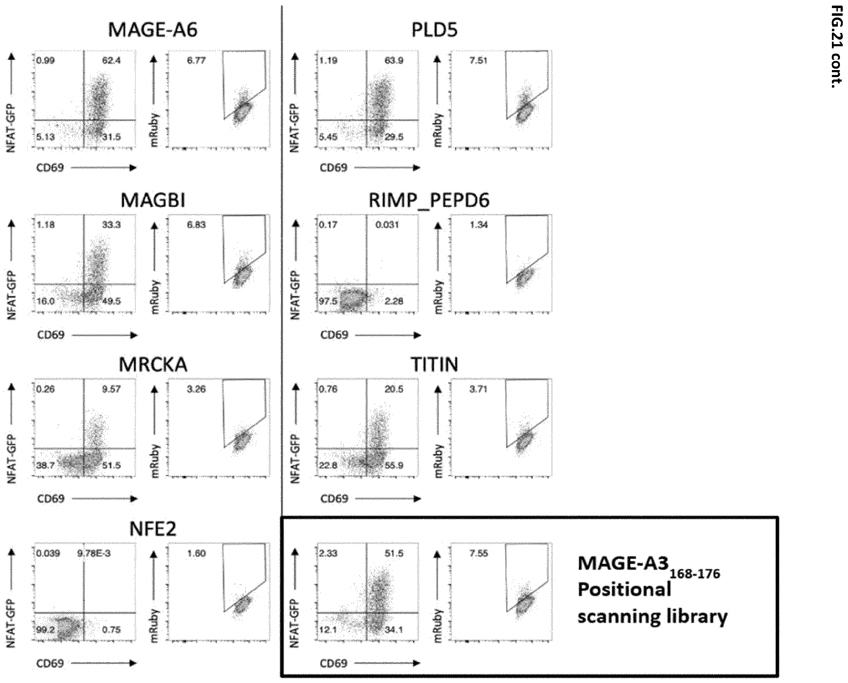

**FIG.21****:** Genomically-encoded candidate T cell antigens and positional scanning libraries induce robust TnT-TCR and ACDC-Ag activation. TnT cells displaying TCRa3a recognising the MAGE-A3₁₆₈₋₁₇₆ EVDPIGHLY T cell antigen (SEQ ID NO: 12) were co-cultured overnight with ACDC-Ag cells expressing HLA-A^{∗}0101 and one of fifteen candidate T cell antigens, including MAGE-A3₁₆₈₋₁₇₆ EVDPIGHLY (SEQ ID NO: 12). In addition, TnT-TCRa3a cells were co-cultured overnight with ACDC-Ag cells expressing HLA-A^{∗}0101 and a positional scanning library of the MAGE-A3₁₆₈₋₁₇₆ EVDPIGHLY T cell antigen (SEQ ID NO: 12) (bottom right). Flow cytometry analysis shows activation of TnT-TCR (NFAT-GFP and CD69; left hand side plots) and ACDC-Ag cells (STAT5-mRuby2; right hand side plots).

### EXAMPLES

### Example 1: Generation of a reporter cell line for display of transgenic TCR antigens and HLA alleles

In order to generate the ACDC cell line, the inventors started with a CRISPR-edited monoclonal derivative of HEK293-Blue cells (IL-2R-positive, JAK3-positive, STAT5-SEAP soluble reporter; Invivogen, US) engineered for constitutive Cas9-GFP expression (CCR5 locus) and introduction of a fluorescent reporter of IL-2 signalling (STAT5-mRuby2 fluorescent reporter). These cells, referred to as pre-ACDC cells hereafter, were subjected to multiple additional steps of CRISPR-Cas9 genome editing in order to facilitate the functional display of transgenes encoding HLA alleles and T cell antigens. The genomic modifications performed for this purpose are summarised in Fig. 3 and described in detail in the sections below. Crucially, ACDC cells and their derivatives were designed to be compatible with the TnT functional TCR display platform (WO 2021/074249). TnT-TCR cells recognising cognate antigen presented by ACDC cells become activated and secrete IL-2. In turn, ACDC cells at the immune synapse sense locally secreted IL-2, leading to the expression of the mRuby2 fluorescent reporter under the control of STATS response element **(****Fig. 4**). In this manner, the identity of TCR-activating antigens can be obtained by performing multiple rounds of fluorescence-activated cell sorting (FACS) coupled with sequencing of the transgenic T cell antigen locus of ACDC cells.

### Example 2: Disruption of endogenous HLA class I expression

The ACDC platform is designed to display TCR antigens presented by HLA class I as these represent the typical targets of engineered TCR-T cell therapies mediating direct cytotoxicity of tumour cells. In order to ensure exclusive expression of *transgenic* HLA alleles, the first step in the development of the ACDC platform was to perform CRISPR-targeted knockout of *endogenous* HLA class I genes. Classical HLA class I genes (Chr 6: HLA-A, HLA-B and, HLA-C, six alleles per individual) are highly polymorphic (> 9,000 allelic variants) but nevertheless possess short stretches of highly conserved DNA sequences (Lank et al., BMC Genomics. 2012 Aug 6;13:378). We took advantage of this feature to design a pan-HLA-I gRNA targeting exon 4 of HLA class I, which was delivered to pre-ACDC cells by means of electroporation. Since pre-ACDC cells possess constitutive Cas9 expression, no exogenous Cas9 was required to observe highly efficient rates of CRISPR-targeted disruption of HLA-A, -B and, -C gene expression (> 95% knockout), as assessed by flow cytometry **(****Fig. 5**). HLA-A/B/C-negative cells were subjected to two rounds of FACS enrichment in order to obtain pure populations of cells lacking expression of endogenous HLA class I alleles, which are referred to as ACDC cells hereafter.

### Example 3: Reconstitution with transgenic HLA class I alleles

In the next step of genome engineering, the inventors sought to replace the GFP transgene present at the CCR5 genomic locus of ACDC cells with a transgene encoding the commonly occurring HLA-A^{∗}0201 allele. To achieve this, ACDC cells were electroporated with a gRNA targeting GFP and a double-stranded DNA homology-directed repair (HDR) template containing the following elements: (i) left and right homology arms of 796 and 935 bp, (ii) HLA-A^{∗}0201 open reading frame, and (iii) SV40 terminator **(****Fig. 6a**). Following transfection and expansion in culture, a monoclonal cell line was derived by single-cell FACS and both genotyped (genomic PCR) and phenotyped (flow cytometry) to confirm CRISPR-targeted replacement of GFP with HLA-A^{∗}0201 at the CCR5 locus (**Fig. 6b**). Resulting cells expressing Cas9 and transgenic HLA-A^{∗}0201, and harbouring a STAT5-mRuby2 reporter of IL-2 signalling are referred to as ACDC-HLA cells (see **Fig. 3**). Using the same genome editing procedure the inventors successfully generated multiple ACDC-HLA cells expressing 18 different HLA class I alleles, thus highlighting the applicability of the ACDC platform for TCR alloreactivity screening assays **(****Figs. 7-9** and **Example 11).**

### Example 4: Validation of ACDC platform functionality following co-culture with TnT-TCR cells

In order to assess the functionality of the ACDC platform, the inventors performed co-culture experiments with TCR-accepting T cells (TnT) expressing a model tumour-targeting TCR, namely TCR_{1G4} recognising the NY-ESO-1₁₅₇₋₁₆₅ peptide SLLMWITQC (SEQ ID NO: 10) presented on HLA-A^{∗}0201. Accordingly, TnT-TCR_{1G4} cells were co-cultured overnight with ACDC-HLA cells (A^{∗}0201) that had been previously pulsed with the SLLMWITQC peptide (SEQ ID NO: 10) or left untreated. Consistent with recognition of cognate antigen, TnT-TCR_{1G4} displayed robust activation following co-culture with peptide-pulsed ACDC-HLA cells, as assessed by detection of the NFAT-GFP reporter of TCR signalling and expression of the early T cell activation marker CD69 by means of flow cytometry. In this co-culture, 29% of peptide-pulsed ACDC-HLA cells expressed mRuby2, thus demonstrating that TnT-TCR-secreted IL-2 successfully drives the expression of the STAT5-mRuby2 fluorescent reporter in ACDC-HLA cells **(****Fig. 10a****).** Notably, minimal levels of background expression of all activation markers were observed in both TnT-TCR_{1G4} and ACDC-HLA cells in the absence of peptide pulsing. In a separate experiment, we further validated the ability of TnT-TCR_{1G4} cells to secrete IL-2 in response to peptide-pulsed HEK-293 cells (A^{∗}0201) by means of ELISA **(****Fig. 10b****).** Finally, the inventors applied single-cell FACS to derive a monoclonal ACDC-HLA (A^{∗}0201) cell line with high sensitivity to both recombinant IL-2 (56% mRuby2+ following stimulation) and TnT-TCR_{1G4}-secreted IL-2 (46.7% mRuby2+ following co-culture) (**Fig. 11**).

### Example 5: Disruption of endogenous beta-2-microglobulin expression

An additional CRISPR-Cas9 genome editing step was performed with the aim of facilitating high-throughput functional screening of genomically-encoded antigen libraries. To this end, the inventors sought to generate a cell line in which correct integration of antigen mutagenesis libraries could be readily validated by means of flow cytometry. Accordingly, the inventors targeted beta-2-microglobulin (B2M) for CRISPR-targeted knockout. B2M is a small structural protein expressed by all nucleated cells that associates noncovalently with multiple receptors (including all MHC class I receptors, i.e., all HLA class I in humans) and that is indispensable for their correct assembly and surface expression. As such, disruption of the native B2M gene in ACDC-HLA cells provided the inventors with the opportunity to derive a cell line lacking B2M expression (and consequently HLA surface expression) that could be reconstituted with gene cassettes encoding a B2M transgene and a particular antigen (i.e., a B2M-Ag cassette). This approach enabled the restoration of B2M/HLA surface expression upon CRISPR-targeted integration of genomically-encoded antigen mutagenesis libraries, thereby providing a selectable marker for FACS. Accordingly, ACDC-HLA (A^{∗}0201) cells were electroporated with a B2M-targeting gRNA, expanded in culture and subjected to multiple rounds of FACS in order to obtain a pure population for B2M negative cells. The resulting cells expressing Cas9, harbouring a STAT5-mRuby2 reporter of IL-2 signalling, expressing a unique transgenic HLA allele and lacking expression of native B2M are referred to as ACDC-B2MKO cells hereafter **(****Fig. 12**).

### Example 6: Reconstitution of ACDC-B2MKO cells with transgenic T cell antigens

In order to demonstrate the feasibility of B2M-Ag reconstitution in ACDC-B2MKO cells, the inventors co-transfected them with a gRNA targeting the AAVS1 locus and an HDR template encoding the wild-type B2M gene and NY-ESO-1₁₅₇₋₁₆₅ SLLMWITQC T cell antigen (SEQ ID NO: 10). The HDR template consisted of: (i) left and right homology arms of 804 and 837 base pairs respectively, mapping to the AAVS1 genomic locus; (ii) CMV enhancer; (iii) CMV promoter; (iv) an open reading frame encoding B2M, a P2A peptide, a signal peptide (i.e., ER signal), and the SLLMWITQC antigen (SEQ ID NO: 10); (v) SV40 poly(A) signal **(****Fig. 13a****).** Following transfection, cells were analysed by flow cytometry for surface expression of B2M, revealing an HDR efficiency of approximately 13% **(****Fig. 13a****).** Cells were then expanded in culture and subjected to multiple rounds of FACS enrichment in order to obtain a pure population of B2M-Ag-positive cells, which are referred to as ACDC-Ag cells hereafter **(****Fig. 13b****).** In addition to ACDC-Ag expressing HLA-A^{∗}0201 and NY-ESO-1₁₅₇₋₁₆₅antigen, the inventors further generated ACDC-Ag cells expressing HLA-A^{∗}0101 and a collection of 12 individual genomically-encoded antigens, thus validating our approach for B2M-Ag reconstitution (HDR efficiency range 7% to 31%) **(****Fig. 14****).**

### Example 7: Functional validation of ACDC-Ag expressing genomically-encoded antigens

Following the successful generation of ACDC-Ag (A^{∗}0201/NY-ESO-1₁₅₇₋₁₆₅) cells by means of CRISPR-targeted reconstitution with a B2M-Ag transgene, the inventors proceeded to validate their functionality in co-culture assays. For this purpose, the inventors performed co-cultures of said ACDC-Ag cells with TnT-TCR cells expressing either TCR_{1G4} (recognising NY-ESO-1₁₅₇₋₁₆₅) or TCR_{DMF5} (recognising a different tumour antigen, MART-1₂₇₋₃₅). Co-culture with TnT-TCR_{1G4} but not with TnT-TCR_{DMF5} cells led to robust expression of NFAT-GFP and CD69 in TnT-TCR cells, and of the STAT5-mRuby2 reporter in ACDC-Ag cells **(****Fig. 15****).** Thus, these results validate the successful presentation of genomically-encoded NY-ESO-1₁₅₇₋₁₆₅ TCR antigen by ACDC-Ag, as well as its ability to activate both TnT-TCR and ACDC-Ag cells in an antigen-specific manner. The inventors applied single-cell FACS to derive a monoclonal ACDC-Ag cell line expressing A^{∗}0201/NY-ESO-1₁₅₇₋₁₆₅ that displayed high sensitivity to IL-2 stimulation, namely clone SC7 (data not shown). SC7 displayed a similar ability to activate TnT-TCR_{1G4} cells (and consequently its STAT5-mRuby2 reporter), to ACDC-HLA (A^{∗}0201) cells pulsed with synthetic NY-ESO-1₁₅₇₋₁₆₅ peptide **(****Fig. 16****).** The inventors further validated the functional presentation of genomically-encoded antigens by performing co-cultures of TnT cells expressing the affinity-enhanced TCRₐ₃ₐ (recognising MAGE-A3₁₆₈₋₁₇₆) (Cameron et al., Sci Transl Med. 2013 Aug 7;5(197):197ra103) and ACDC-Ag cells expressing HLA-A^{∗}0101 and a collection of 15 individual candidate T cell antigens **(****Fig. 21****).** Together, the inventor's results validate CRISPR-targeted display of genomically-encoded antigens in the ACDC platform that show activation levels comparable to those induced by the display of synthetic peptides.

### Example 8: Enhancing TCR-Safe sensitivity through CRISPR-targeted integration of an enhanced common gamma chain variant

In order to further enhance the sensitivity of ACDC cells and their derivatives for secreted IL-2, a variant of the IL-2 receptor gamma subunit (i.e., γ_{c}, common gamma chain, or CD132) with enhanced sensitivity to IL-2 signalling, referred to hereafter as eCD132, is used as the selectable marker for CRISPR-targeted integration of T cell antigen libraries. Accordingly, the endogenous CD132 gene in ACDC-HLA cells is subjected to CRISPR-targeted knockout in order to obtain ACDC-CD132-KO cells lacking surface expression of CD132, as assessed by flow cytometry. Next, ACDC-CD132-KO cells are co-transfected with a gRNA targeting the AAVS1 locus and an HDR template encompassing an eCD132-Ag cassette encoding the eCD132 variant and a T cell antigen (or T cell antigen libraries). The HDR template consists of: (i) left and right homology arms of 804 and 837 base pairs respectively, mapping to the AAVS1 genomic locus; (ii) CMV enhancer; (iii) CMV promoter; (iv) an open reading frame encoding eCD132, a P2A peptide, a signal peptide (i.e., ER signal), and a T cell antigen (or T cell antigen library); (v) SV40 poly(A) signal. As such, the use of eCD132 serves three purposes: (i) enhance the sensitivity of the STAT5-mRuby2 reporter system, (ii) act as a selectable marker for the integration of candidate T cell antigen libraries, (iii) replace the use of B2M-Ag cassettes for B2M reconstitution for the use of eCD132-Ag cassettes for CD132 reconstitution.

### Example 9: Application of TCR-Safe for cross-reactivity profiling by high-throughput positional scanning of genomically-encoded antigens

While the use of peptide scanning has been useful for the prediction of potential TCR off-targets in the recent past, this method is both time-consuming (need for multiple individual co-cultures) and expensive (peptide synthesis). As such, positional antigen scanning represents an important application in which TCR-Safe significantly reduces the time (pooled screening) and costs (inexpensive ssDNA oligonucleotide pools) associated with TCR cross-reactivity profiling **(****Fig. 1****).** The inventors first applied TCR-Safe for positional scanning of the MAGE-A3₁₆₈₋₁₇₆ EVDPIGHLY antigen (SEQ ID NO: 12) using ACDC-HLA (A^{∗}0101) cells. For this purpose, a single-site saturation mutagenesis library in which tiled degenerate NNK codons were incorporated at each position of the MAGE-A3₁₆₈₋₁₇₆ EVDPIGHLY antigen (SEQ ID NO: 12) was generated by PCR amplification of a template plasmid containing a B2M-Ag cassette flanked by homology arms flanking the AAVS1 genomic locus **(****Fig. 17a****).** After propagation in E. coli and plasmid purification, HDR templates were generated by PCR and electroporated into ACDC-HLA (A^{∗}0101) cells alongside a gRNA targeting the AAVS1 genomic site. Following expansion in culture, transfected cells were analysed by flow cytometry, revealing successful integration of the positional scanning library as evidenced by restored surface expression of B2M and HLA-A/B/C (HDR efficiency ~ 10%) **(****Fig. 17b****).** Sanger sequencing of transgenic B2MAg PCR amplicons further confirmed successful CRISPR-targeted integration of the MAGE-A3₁₆₈₋₁₇₆ EVDPIGHLY (SEQ ID NO: 12) positional scanning library as indicated by the occurrence of single codon mutants in 7 out of 8 sequenced clones **(****Fig. 17c****).** Following transfection, ACDC-Ag cells are enriched by several rounds of FACS in order to obtain pure populations of B2M-Ag-positive cells. Following enrichment, ACDC-Ag cells are co-cultured with TnT-TCR cells expressing a TCR of interest, for instance a therapeutic TCR candidate **(****Fig. 21****, bottom right).** Following co-culture, ACDC-Ag cells expressing the STAT5-mRuby2 reporter (i.e., indicative of antigen-specific TnT-TCR activation) are enriched by FACS and expanded in culture. The co-culture and FACS enrichment process is repeated several times until highly enriched populations of STAT5-mRuby2-positive cells are obtained. PCR amplification of the transgenic B2M-Ag locus is then performed on the isolated STAT5-mRuby2-positive ACDC-Ag cells, and the resulting amplicons analysed by deep sequencing. Computational analysis of deep sequencing data is applied in order to identify activating single amino acid mutants of the intended target, and crucially, their read enrichment levels relative to their frequencies in the starting pool of B2M-Ag-positive cells. Using this data, antigen motifs at different thresholds of read enrichment are generated and used to interrogate human proteome databases, splice variant databases and single-nucleotide polymorphism databases in order to identify candidate off-targets.

### Example 10: Applications of TCR-Safe for cross-reactivity profiling and target discovery by high-throughput functional screening of combinatorial antigen libraries

A second application of TCR-Safe for TCR cross-reactivity profiling consists in the display and high-throughput functional selection of combinatorial genomically-encoded candidate T cell antigen libraries **(****Fig. 1****).** Combinatorial T cell antigen library designs are broadly divided into those of moderate diversity (10²-10⁴ members) and those of high diversity (>10⁴ members) **(****Fig. 18****).** Moderate diversity T cell antigen libraries include but are not limited to libraries encoding candidate TCR off-targets predicted from positional scanning experiments (see Example 9) and libraries encoding all existing human sequences that are four or less amino acids away from the intended target T cell antigen (or a subset therein). High diversity T cell antigen libraries include but are not limited to libraries mimicking amino acid frequencies of publicly available peptide binding motifs to specific HLA alleles, and those encompassing the entire human proteome with gene constructs encoding long overlapping sequences (e.g., 30 - 100 codons, with 30-50 codon overlap). After library design, assembly and CRISPR-targeted integration into ACDC-HLA cells, cells enriched for B2M-Ag display are co-cultured with TnT-TCR cells expressing a TCR of interest, such as an engineered therapeuticTCR candidate. ACDC-Ag cells displaying antigens recognised by TnT-TCR are stimulated by secreted IL-2 to express the STAT5-mRuby2 fluorescence reporter, and subjected to FACS enrichment. Several rounds of co-culture and FACS enrichment of STAT5-mRuby2-positive cells are performed in order to obtain pure populations of ACDC-Ag cells harbouring TCR-activating antigens (positive training data), as well as for the iterative isolation STAT5-mRuby2-negative cells (negative training data). Deep sequencing of PCR amplicons encompassing the transgenic B2M-Ag locus is next performed on every selected and enriched ACDC-Ag fraction and sequencing data is analysed computationally to determine the read frequency enrichment levels of library members across selection steps to identify putative TCR antigens. In order to expand the limits of experimental T cell antigen screening, read frequency enrichment data from STAT5-mRuby2-positive and STAT5-mRuby2-negative fractions is encoded (e.g., one-hot encoding) and used to train supervised machine learning models (e.g. SVM, Random Forest, neural networks) that can perform classification of the screened TCR for activation or non-activation based on a given T cell antigen sequence. These models are then applied to the entire human peptidome in order to assign every candidate antigen sequence with a probability of TCR activation, thus enabling comprehensive discovery of T cell antigens that activate the candidate TCR. A related application of TCR-Safe is the discovery of T cell antigens recognised by TCRs of biological significance (e.g., tumour-reactive) but with unknown specificity (i.e., orphan TCRs). For example, candidate tumour-specific TCRs are identified by single-cell TCR repertoire and transcriptomic profiling of tumour-infiltrating lymphocytes or longitudinal PBMC samples obtained from patients responding to immune checkpoint blockade therapy. Once identified, TCR candidates are displayed on TnT cells by means of CRISPR-targeted integration and subjected to the TCR-Safe protocol (described above) in order to discover or predict their targeted T cell antigens.

### Example 11: Application of TCR-Safe for high-throughput functional HLA alloreactivity screening

An important application of TCR-Safe for alloreactivity profiling consists in the display of HLA class I allele libraries and their functional screening at high-throughput **(****Fig. 2****).** In this context, TCR-Safe enables the screening of a collection of commonly occurring HLA class I alleles (HLA-A = 104 alleles, HLA-B = 63 alleles, HLA-C = 28 alleles) in a pooled fashion. This collection of HLA alleles has a cumulative allele frequency of more than 99% for each HLA-A, HLA-B and HLA-C genes across multiple ethnicities, thus providing comprehensive coverage of the human population as a whole (Gonzalez-Galarza et al., Nucleic Acids Res. 2020 Jan 8;48(D1):D783-D788). Notably, a similar range of HLA allele coverage is typically achieved with 50 or more individual B-LCL lines (Sanderson et al., Oncoimmunology. 2019 Nov 24;9(1):1682381), thus highlighting an important advantage of TCR-Safe pooled alloreactivity screening in terms of throughput. Once designed, gene cassettes encoding individual HLA alleles are generated by gene synthesis (Twist Biosciences) and cloned as a pool into a plasmid containing a 5' homology arm mapping to the region upstream of the GFP transgene and a 3' homology arm mapping to the CCR5 locus in ACDC cells **(****Fig. 19** and *'Reconstitution with transgenic HLA class I alleles'* section). HDR templates are then generated by PCR and the resulting amplicons electroporated into ACDC cells alongside a gRNA targeting the GFP transgene for CRISPR-targeted reconstitution with HLA allele libraries **(****Fig. 20a****).** Next, ACDC-HLA cells are subjected to multiple rounds of FACS enrichment in order to obtain pure populations of B2M-positive HLA-A/B/C-positive cells **(****Fig. 20b****).** Following enrichment, ACDC-HLA cells are used in two types of co-cultures with TnT-TCR cells, namely (i) co-culture with TnT cells expressing a single TCR of interest; and (ii) co-culture with TnT-TCR cells displaying a library of engineered TCRs. In both settings, alloreactive activation of TnT-TCR cells leads to IL-2 secretion and subsequent expression of the STAT5-mRuby in ACDC-HLA cells, as well as expression of NFAT-GFP and CD69 in TnT-TCR cells. STAT5-mRuby2-positive ACDC-HLA cells are enriched by FACS and their transgenic HLA locus sequenced by deep sequencing, and activated TnT-TCR cells are similarly enriched by FACS for deep sequencing of their transgenic TCR locus. As such, computational analysis of read frequency enrichment across selection steps provides key information on the identity of both alloreactive TCRs and the HLA alleles eliciting the observed alloreactivity.

### Materials and Methods

### Cell Culture

ACDC cells and their derivatives were cultured in DMEM medium (ATCC, #30-2002) with 10 % FBS (gibco, #16000-044) and 1 % Penicillin-Streptomycin (Gibco, #15140-122). For prolonged storage, cells were frozen in Bambanker freezing medium (GCLTEC, #BBD01) and stored in liquid nitrogen.

### CRISPR-Cas9 Genome Editing

HDR templates were generated by PCR amplification followed by column-purification using the DNA Clean & Concentrator-25 kit (Zymo Research, D4005). Cell transfection was performed using electroporation (SF Cell Line Solution Box, Lonza, #V4XC-2024) with the "HEK-293" protocol on the 4D-Nucleofector (Lonza). For genome editing, 200 µM tracrRNA and 200 µM crRNA were mixed at equimolar concentration to form the gRNA complex. For HDR, 1 µg PCR-amplified DNA constructs and 8 µL gRNA were used per transfection. After one week, HDR efficiency was assessed by flow cytometry. Transfections were either bulk or single cell sorted, expanded and characterised by flow cytometry, PCR, Sanger, or next-generation sequencing.

### IL-2 stimulation assay

ACDC cells and their derivatives were stimulated for 24 hours at a density of 5 x 10^5 / mL with 20 ng/mL monomeric human IL-2 (Peprotech, 200-02). The cells were washed with ice-cold FACS buffer (PBS supplemented with 2% FBS) and resuspended in 120 uL FACS buffer before scanning for mRuby2 expression by flow cytometry.

### Molecular cloning of B2M-Ag gene cassettes

B2M-Ag inserts and pMT2-PL vector containing AAVS1 safe harbour homology arms under control of the CMV promoter were digested with Kpnl and Xbal for 1 h at 37C. Vector 5' ends were dephosphorylated by addition of 1 µL calf intestinal phosphatase (CIP) for a further 30 min. The digested vector and inserts were gel-purified (ZymoClean Gel DNA Recovery Kit, Zymo Research, #D4001) and ligated using T4 ligase (NEB, #M0202F) at a molar ratio 3:1 insert-to-vector for 2h at RT. Ligation mixes were transformed into NEB5α bacteria (NEB, #C2988J) according to the manufacturer's instructions. Successful cloning was validated by Sanger sequencing of individual bacterial transformants.

### Generation of deep mutational scanning (DMS) libraries

Site-specific mutagenesis was used for library construction. The primers were designed and purchased as (i) a pool of reverse primer containing tiled NNK degenerate codons across the sequence encoding the target T cell antigen (IDT), and (ii) the forward primer with 5' phosphorylated is designed to that the 5' ends of the two primers anneal back-to- back. 30 nmol of each reverse primer and 300 nmol of forward primer were mixed and subjected to PCR amplification (KAPA Biosystems, #KK2101) on pMT2-B2M-Ag plasmid as described. T4 ligase (NEB, #M0202F) was used for circularization of the PCR product for 2 h at RT. Ligation mixes were transformed into NEB5α bacteria (NEB, #C2988J) according to the manufacturer's instructions. Each transformation mix was plated on ampicillin LB agar in Square Bioassay dishes (Corning, #431111). 1/100 and 1/1000 dilutions of the transformations were also prepared and plated on ampicillin plates to determine the number of transformants the following day. Colonies from the Square Bioassay plates were collected by adding 15 mL of LB media to each plate and resuspending. Colonies from each library were pooled together in a single tube and centrifuged at 4800 g for 10 min. The supernatant was discarded and the plasmid DNA was isolated following the Midiprep protocol (Zymo Research, # D4201).

### crRNA sequences

CRISPR-RNA (crRNA) reagents targeting the CCR5 (5'-TGACATCAATTATTATACAT-3') (SEQ ID NO:57), eGFP (5'-CAACTACAAGACCCGCGCCG-3') (SEQ ID NO:58), AAVS1 (5'-GGGGCCACTAGGGACAGGAT-3') (SEQ ID NO:59), HLA-A/B/C (5'-CTGCGGAGATCACACTGACC-3') (SEQ ID NO:1) and B2M (5'-AAGTCAACTTCAATGTCGGA-3') (SEQ ID NO:60) loci were purchased from IDT. The sequences above reflect the DNA template used to design the crRNA constructs.

### Genotyping of ACDC clones

Genomic DNA was extracted using the QuickExtract solution kit (Lucigen, #0905T). Genomic and RT-PCR were performed using the Q5^{®} High-Fidelity 2X Master Mix (NEB, #M0492S). See appendix for primer sequences.

### Flow Cytometry and Cell Sorting

For flow cytometric analysis of surface antigens, cells were washed with FACS buffer and stained with appropriate fluorophore-conjugated antibodies (all BioLegend: B2M-PE (#316306), HLA-A,B,C-APC (#311410), HLA-A2-APC (#343308), CD69-PE/Cy7 (#310912), for 20 min at 4C. Cells were washed twice in the FACS buffer, and fluorescence was measured using the Cytoflex S (Beckman Coulter) flow cytometer. FACS was performed either on the Aria III instrument (BD Biosciences) or FACSMelody (BD Bioscience).

**ST.26 SEQUENCE LISTING - HUMAN READABLE FORMAT FOR INTERNAL USE**

| Invention title | | CELL LINE FOR DISCOVERING TCR |
|---|---|---|
| ANTIGENS AND USES THEREOF (en) | | |
| First applicant name | | ETH Zurich (en) |
| First applicant name (Latin) | | - |
| First inventor name | | - |
| First inventor name (Latin) | | - |
| Applicant file reference | | AE2865 EP BS |
| Application number | | Filing Date |
| | | |
| Earliest priority number | | - |
| Earliest priority date | | - |
| DTD version | | V1_3 |
| File name | | AE2865 EP BS.xml |
| Software name | | WIPO Sequence |
| Software version | | 2.0.0 |
| Production date | | 2022-07-01 |
| Original free text language code | | - |
| Non English free text language code | | - |
| **Sequence total quantity** | | **60** |
| SEQ ID NO: | 1 | |
| length | 20 | |
| molecule type | RNA | |
| division | PAT | |
| Key | Location/Qualifiers | |
| source | 1..20 | |
| | /mol_type=other RNA | |
| | /organism=synthetic construct | |
| Sequence | | |
| ctgcggagat cacactgacc | 20 | |
| // | | |
| SEQ ID NO: | 2 | |
| length | 17 | |
| molecule type | DNA | |
| division | PAT | |
| Key | Location/Qualifiers | |
| source | 1..17 | |
| | /mol_type=other DNA | |
| | /organism=synthetic construct | |
| Sequence | | |
| ggttttcctg gaaagtt | 17 | |
| // | | |
| SEQ ID NO: | 3 | |
| length | 15 | |
| molecule type | DNA | |
| division | PAT | |
| Key | Location/Qualifiers | |
| source | 1..15 | |
| | /mol_type=other DNA | |
| | /organism=synthetic construct | |
| Sequence | | |
| agttctgaga aaagt | 15 | |
| // | | |
| SEQ ID NO: | 4 | |
| length | 347 | |
| molecule type | AA | |
| division | PAT | |
| Key | Location/Qualifiers | |
| source | 1..347 | |
| | /mol_type=protein | |
| | /organism=Homo sapiens | |
| Sequence | | |
| LNTTILTPNG NEDTTADFFL TTMPTDSLSV STLPLPEVQC FVFNVEYMNC TWNSSSEPQP 60 TNLTLHYWYK NSDNDKVQKC SHYLFSEEIT SGCQLQKKEI HLYQTFVVQL QDPREPRRQA 120 TQMLKLQNLV IPWAPENLTL HKLSESQLEL NWNNRFLNHC LEHLVQYRTD WDHSWTEQSV 180 DYRHKFSLPS VDGQKRYTFR VRSRFNPLCG SAQHWSEWSH PIHWGSNTSK ENPFLFALEA 240 VVISVGSMGL IISLLCVYFW LERTMPRIPT LKNLEDLVTE YHGNFSAWSG VSKGLAESLQ 300 PDYSERLCLV SEIPPKGGAL GEGPGASPCN QHSPYWAPPC YTLKPET 347 | | |
| // | | |
| SEQ ID NO: | 5 | |
| length | 18 | |
| molecule type | AA | |
| division | PAT | |
| Key | Location/Qualifiers | |
| source | 1..18 | |
| | /mol_type=protein | |
| | /organism=synthetic construct | |
| Sequence | | |
| EGRGSLLTCG DVEENPGP | 18 | |
| // | | |
| SEQ ID NO: | 6 | |
| length | 19 | |
| molecule type | AA | |
| division | PAT | |
| Key | Location/Qualifiers | |
| source | 1..19 | |
| | /mol_type=protein | |
| | /organism=synthetic construct | |
| Sequence | | |
| ATNFSLLKQA GDVEENPGP | 19 | |
| // | | |
| SEQ ID NO: | 7 | |
| length | 20 | |
| molecule type | AA | |
| division | PAT | |
| Key | Location/Qualifiers | |
| source | 1..20 | |
| | /mol_type=protein | |
| | /organism=synthetic construct | |
| Sequence | | |
| QCTNYALLKL AGDVESNPGP | 20 | |
| // | | |
| SEQ ID NO: | 8 | |
| length | 22 | |
| molecule type | AA | |
| division | PAT | |
| Key | Location/Qualifiers | |
| source | 1..22 | |
| | /mol_type=protein | |
| | /organism=synthetic construct | |
| Sequence | | |
| VKQTLNFDLL KLAGDVESNP | GP 22 | |
| // | | |
| SEQ ID NO: | 9 | |
| length | 20 | |
| molecule type | AA | |
| division | PAT | |
| Key | Location/Qualifiers | |
| source | 1..20 | |
| | /mol_type=protein | |
| | /organism=Homo sapiens | |
| Sequence | | |
| MYRMQLLSCI ALSLALVTNS | 20 | |
| // | | |
| | | |
| SEQ ID NO: | 10 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| SLLMWITQC | 9 | |
| // | | |
| SEQ ID NO: | 11 | |
| length | 10 | |
| molecule type | AA | |
| division | PAT | |
| Key | Location/Qualifiers | |
| source | 1..10 | |
| | /mol_type=protein | |
| | /organism=synthetic construct | |
| Sequence | | |
| ELAGIGILTV | 10 | |
| // | | |
| SEQ ID NO: | 12 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVDPIGHLY | 9 | |
| // | | |
| SEQ ID NO: | 13 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| XVDPIGHLY | 9 | |
| // | | |
| SEQ ID NO: | 14 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EXDPIGHLY | 9 | |
| // | | |
| SEQ ID NO: | 15 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVXPIGHLY | 9 | |
| // | | |
| SEQ ID NO: | 16 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVDXIGHLY | 9 | |
| // | | |
| SEQ ID NO: | 17 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVDPXGHLY | 9 | |
| // | | |
| SEQ ID NO: | 18 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVDPIXHLY | 9 | |
| // | | |
| SEQ ID NO: | 19 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence EVDPIGXLY // | 9 | |
| SEQ ID NO: | 20 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVDPIGHXY | 9 | |
| // | | |
| SEQ ID NO: | 21 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVDPIGHLX | 9 | |
| // | | |
| SEQ ID NO: | 22 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| gaagtggacc ccatcggcca cttgtac | 27 | |
| // | | |
| SEQ ID NO: | 23 | |
| length | 27 | |
| molecule type | DNA | |
| division | PAT | |
| Key | Location/Qualifiers | |
| source | 1..27 | |
| | /moltype=other DNA | |
| Sequence | _ /organism=synthetic construct | |
| nnkgtggacc ccatcggcca cttgtac | 27 | |
| // | | |
| SEQ ID NO: | 24 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| gaannkgacc ccatcggcca cttgtac | 27 | |
| // | | |
| SEQ ID NO: | 25 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| gaagtgnnkc ccatcggcca cttgtac | 27 | |
| // | | |
| SEQ ID NO: | 26 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| gaagtggacn nkatcggcca cttgtac | 27 | |
| // | | |
| SEQ ID NO: | 27 | |
| length | 27 | |
| molecule type | DNA | |
| division | PAT | |
| Key | Location/Qualifiers | |
| source | 1..27 | |
| | /mol_type=other DNA | |
| Sequence | /organism=synthetic construct | |
| gaagtggacc ccnnkggcca cttgtac | 27 | |
| // | | |
| SEQ ID NO: | 28 | |
| length | 27 | |
| molecule type | DNA | |
| division | PAT | |
| Key | Location/Qualifiers | |
| source | 1..27 | |
| | /moltype=other DNA | |
| | _ /organism=synthetic construct | |
| Sequence | | |
| gaagtggacc ccatcnnkca cttgtac | 27 | |
| // | | |
| SEQ ID NO: | 29 | |
| length | 27 | |
| molecule type | DNA | |
| division | PAT | |
| Key | Location/Qualifiers | |
| source | 1..27 | |
| | /moltype=other DNA | |
| | _ /organism=synthetic construct | |
| Sequence | | |
| gaagtggacc ccatcggcnn kttgtac | 27 | |
| // | | |
| SEQ ID NO: | 30 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| gaagtggacc ccatcggcca cnnktac | 27 | |
| // | | |
| SEQ ID NO: | 31 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| gaagtggacc ccatcggcca cttgnnk | 27 | |
| // | | |
| SEQ ID NO: | 32 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVDPIIHLY | 9 | |
| // | | |
| SEQ ID NO: | 33 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVDPIGHMY | 9 | |
| // | | |
| SEQ ID NO: | 34 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVDRIGHLY | 9 | |
| // | | |
| SEQ ID NO: | 35 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVDPISHLY | 9 | |
| // | | |
| SEQ ID NO: | 36 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVDPIGHLN | 9 | |
| // | | |
| SEQ ID NO: | 37 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| EVTPIGHLY | 9 | |
| // | | |
| SEQ ID NO: | 38 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| XLLMWITQC | 9 | |
| // | | |
| SEQ ID NO: | 39 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| SXLMWITQC | 9 | |
| // | | |
| SEQ ID NO: | 40 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| SLXMWITQC | 9 | |
| // | | |
| SEQ ID NO: | 41 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| SLLXWITQC | 9 | |
| // | | |
| SEQ ID NO: | 42 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| SLLMXITQC | 9 | |
| // | | |
| SEQ ID NO: | 43 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| SLLMWXTQC | 9 | |
| // | | |
| **SEQ** ID NO: | 44 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| SLLMWIXQC | 9 | |
| // | | |
| SEQ ID NO: | 45 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| SLLMWITXC | 9 | |
| // | | |
| SEQ ID NO: | 46 | |
| length | 9 | |
| molecule type | AA | |
| division | PAT | |
| Key | Location/Qualifiers | |
| source | 1..9 | |
| | /mol_type=protein | |
| | /organism=synthetic construct | |
| Sequence | | |
| SLLMWITQX | 9 | |
| // | | |
| SEQ ID NO: | 47 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| tcactgctga tgtggatcac acaatgc | 27 | |
| // | | |
| SEQ ID NO: | 48 | |
| length | 27 | |
| molecule type | DNA | |
| division | PAT | |
| Key | Location/Qualifiers | |
| source | 1..27 | |
| | /moltype=other DNA | |
| | _ /organism=synthetic construct | |
| Sequence | | |
| nnkctgctga tgtggatcac acaatgc | 27 | |
| // | | |
| SEQ ID NO: | 49 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| tcannkctga tgtggatcac acaatgc | 27 | |
| // | | |
| SEQ ID NO: | 50 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| tcactgnnka tgtggatcac acaatgc | 27 | |
| // | | |
| SEQ ID NO: | 51 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| tcactgctgn nktggatcac acaatgc | 27 | |
| // | | |
| SEQ ID NO: | 52 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| tcactgctga tgnnkatcac acaatgc | 27 | |
| // | | |
| SEQ ID NO: | 53 | |
| length | 27 | |
| molecule type | DNA | |
| division | PAT | |
| Key | Location/Qualifiers | |
| source | 1..27 | |
| | /moltype=other DNA | |
| | _ /organism=synthetic construct | |
| Sequence | | |
| tcactgctga tgtggnnkac acaatgc | 27 | |
| // | | |
| SEQ ID NO: | 54 | |
| length | 27 | |
| molecule type | DNA | |
| division | PAT | |
| Key | Location/Qualifiers | |
| source | 1..27 | |
| | /moltype=other DNA | |
| | _ /organism=synthetic construct | |
| Sequence | | |
| tcactgctga tgtggatcnn kcaatgc | 27 | |
| // | | |
| SEQ ID NO: | 55 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| tcactgctga tgtggatcac annktgc | 27 | |
| // | | |
| SEQ ID NO: | 56 | |
| length | 27 | |
| molecule type | DNA | |
| division | PAT | |
| Key | Location/Qualifiers | |
| source | 1..27 | |
| | /mol_type=other DNA | |
| | /organism=synthetic construct | |
| Sequence | | |
| tcactgctga tgtggatcac | acaannk 27 | |
| // | | |
| SEQ ID NO: | 57 | |
| length | 20 | |
| molecule type | RNA | |
| division | PAT | |
| Key | Location/Qualifiers | |
| source | 1..20 | |
| | /mol_type=other RNA | |
| | /organism=synthetic construct | |
| Sequence | | |
| tgacatcaat tattatacat | 20 | |
| // | | |
| SEQ ID NO: | 58 | |
| length | 20 | |
| molecule type | RNA | |
| division | PAT | |
| Key | Location/Qualifiers | |
| source | 1..20 | |
| | /mol_type=other RNA | |
| | /organism=synthetic construct | |
| Sequence | | |
| caactacaag acccgcgccg | 20 | |
| // | | |
| SEQ ID NO: | 59 | |
| length | 20 | |
| molecule type | RNA | |
| division | PAT | |
| Key | Location/Qualifiers | |
| source | 1..20 | |
| | /moltype=other RNA | |
| | _ /organism=synthetic construct | |
| Sequence | | |
| ggggccacta gggacaggat | 20 | |
| // | | |
| SEQ ID NO: | 60 | |
| length | 20 | |
| molecule type | RNA | |
| division | PAT | |
| Key | Location/Qualifiers | |
| source | 1..20 | |
| | /mol_type=other RNA | |
| | /organism=synthetic construct | |
| Sequence | | |
| aagtcaactt caatgtcgga | 20 | |
| // | | |

## Claims

1. A cell line wherein the endogenous class I and/or class II HLA alleles are inactivated, the cell line further comprising
a) a polynucleotide encoding a first fluorescent marker under control of at least one STAT response element, and
b) an interleukin 2 (IL-2) receptor.

2. The cell line according to claim 1, wherein the polynucleotide encoding the first fluorescent marker is under control of at least 2, 3, 4 or 5 STAT response elements; and/or wherein the IL-2 receptor is an engineered IL-2 receptor, in particular wherein the engineered IL-2 receptor comprises an engineered common gamma chain.

3. The cell line according to claim 1 or 2, wherein the cell line further comprises a heterologous polynucleotide encoding an HLA allele,
in particular wherein the endogenous class I HLA alleles are inactivated in said cell line and wherein the cell line comprises a heterologous polynucleotide encoding a class I HLA allele

4. The cell line according to any one of claims 1 to 3, wherein the endogenous gene encoding beta-2 microglobulin is inactivated in said cell line,
and preferably wherein the cell line further comprises a heterologous polynucleotide encoding a beta-2 microglobulin.

5. The cell line according to any one of claims 1 to 4, wherein the cell line further comprises a polynucleotide encoding a peptide, preferably wherein the polynucleotide encoding the beta-2 microglobulin and the polynucleotide encoding the peptide are transcriptionally fused, in particular wherein the polynucleotide encoding the beta-2 microglobulin and the polynucleotide encoding the peptide are fused via a linker, in particular wherein the linker encodes a protease-specific cleavage site and/or a self-cleaving peptide, in particular wherein the peptide further comprises a signal peptide, in particular wherein the peptide is an MHC class I peptide.

6. The cell line according to any one of claims 1 to 5, wherein the cell line further comprises a polynucleotide encoding a sequence-specific endonuclease, in particular wherein the sequence-specific endonuclease is a CRISPR-associated (Cas) protein, in particular wherein the CRISPR-associated (Cas) protein is Cas9.

7. A method for identifying potential off-targets of a T cell receptor (TCR), the method comprising the steps of:
a) providing a plurality of cells according to claim 5, wherein at least two cells comprised in the plurality of cells encode a different peptide variant that has been obtained by mutagenesis of a known antigenic peptide;
b) contacting the plurality of cells of step (a) with a plurality of T cells encoding a TCR that is specific for said known antigenic peptide;
c) isolating cells that express the first fluorescent marker, in particular wherein the cells that express the first fluorescent marker are isolated by fluorescence-activated cell sorting (FACS).;
d) sequencing the polynucleotides encoding the peptide variants in the cells that have been isolated in step ^{©}; and
e) identifying potential off-targets of the TCR of interest based on the sequencing results that have been obtained in step (d).

8. The method according to claim 7, wherein the plurality of cells encode at least 5, 10, 20, 50, 100, 200, 300, 500 or 1000 different peptide variants that have been obtained by mutagenesis of a known antigenic peptide; and/or wherein the peptide variants have been obtained by site-directed mutagenesis of the known antigenic peptide, in particular by site-directed saturation mutagenesis of the known antigenic peptide.

9. The method according to any one of claims 14 to 17, wherein the polynucleotides encoding the peptide variants are sequenced by Sanger sequencing or wherein the polynucleotides encoding the peptide variants are sequenced by deep sequencing, in particular wherein potential off-targets are identified by read enrichment analysis of the deep sequencing results;
optionally wherein the method comprises an additional step of querying a potential off-target of a TCR that has been identified in step (e) against a protein database.

10. A method for identifying a target of a T cell receptor (TCR) of interest, the method comprising the steps of:
a) providing a plurality of cells according to claim 5, wherein at least two cells comprised in the plurality of cells encode a different peptide candidate;
b) contacting the plurality of cells of step (a) with a plurality of T cells encoding a TCR of interest;
c) isolating cells that express the first fluorescent marker, in particular wherein the cells that express the first fluorescent marker are isolated by fluorescence-activated cell sorting (FACS);
d) sequencing the nucleic acids encoding the peptide candidates in the cells that have been isolated in step (c); and
e) identifying a target of the TCR of interest based on the sequencing results that have been obtained in step (d).

11. The method according to claim 10, wherein the polynucleotides encoding the peptide candidates are sequenced by Sanger sequencing or wherein the polynucleotides encoding the peptide candidates are sequenced by deep sequencing,
in particular wherein potential targets of the TCR of interest are identified by read enrichment analysis of the deep sequencing results;
optionally wherein the method comprises a further step of predicting targets of the TCR of interest by applying a machine learning model to a human peptidome database, wherein the machine learning model has been trained with the deep sequencing data.

12. A method for assessing the alloreactivity of a T cell receptor (TCR), the method comprising the steps of:
a) providing a plurality of cells according to claim 3, wherein the plurality of cells encode at least one heterologous HLA allele;
b) contacting the plurality of cells of step (a) with a plurality of T cells;
c) isolating cells that express the first fluorescent marker, in particular wherein the cells that express the first fluorescent marker are isolated by fluorescence-activated cell sorting (FACS);
d) sequencing the heterologous polynucleotides encoding the HLA alleles in the cells that have been isolated in step (c); and
e) identifying an HLA molecule as a target of an alloreactive TCR based on the sequencing results that have been obtained in step (d).

13. The method according to claim 12, wherein at least two cells in the plurality of cells of step (a) encode a different HLA allele, in particular wherein at least 5, 10, 25, 50, 75, 100, 150, 200, 300, 400, 500, 1'000, 2'500, 5'000, 10'000 or 25'000 cells in the plurality of cells of step (a) encode a different HLA allele; and/or
wherein the T cells express an identical TCR or wherein at least two T cells in the plurality of T cells express different TCRs, in particular wherein at least 5, 10, 20, 30, 40, 50, 60, 70, 80, 90 or 100 T cells in the plurality of T cells express different TCRs.

14. The method according to claim 12 or 13, wherein the T cell is an engineered T cell, in particular wherein the engineered T cell comprises a polynucleotide encoding a second fluorescent marker under control of an NFAT transcription factor;
in particular wherein the method comprising further steps of
f) isolating T cells that express the second fluorescent marker, in particular wherein the cells that express the second fluorescent marker, are isolated by fluorescence-activated cell sorting (FACS);
g) sequencing the polynucleotides encoding the TCRs in T cells that have been isolated in step (f); and
h) identifying a TCR as an alloreactive TCR based on the sequencing results that have been obtained in step (g).

15. The method according to any one of claims 12 to 14, wherein the polynucleotides encoding the HLA alleles, and optionally the TCRs, are sequenced by Sanger sequencing or wherein the polynucleotides encoding the HLA alleles, and optionally the TCRs, are sequenced by deep sequencing,
in particular wherein HLA molecules are identified as a target of an alloreactive TCR, and/or wherein TCRs are identified as alloreactive TCRs by read enrichment analysis of the deep sequencing results.
